# EUROPEAN PATENT APPLICATION

(11) **EP 2 742 948 A1**
(43) Date of publication of application: **18.06.2014**
(21) Application number: 12772116.5
(22) Date of filing: 10.08.2012
(51) Int. Cl.: A61K 38/19, A61P 39/02

(54) **USE OF CARDIOTROPHIN-1 FOR THE TREATMENT OF KIDNEY DISEASES**

(30) Priority: 10.08.2011 ES 201100933 P
(71) Applicant: Digna Biotech, S.L., 31008 Pamplona Navarra (ES); Universidad de Salamanca, 37008 Salamanca (ES)
(72) Inventor: GARCÍA CENADOR, Begoña, E-31008 Pamplona (ES); GARCÍA CRIADO, Javier, E-31008 Pamplona (ES); LÓPEZ HERNÁNDEZ, Francisco Javier, E-31008 Pamplona (ES); LÓPEZ NOVOA, Jose Miguel, E-31008 Pamplona (ES); PEREZ DE OBANOS MARTELL, María Pilar, E-31008 Pamplona (ES); RUIZ ECHEVERRÍA, Juan, E-31008 Pamplona (ES)
(74) Representative: ABG Patentes, S.L.
(86) International application number: PCT/ES2012/070624
(87) International publication number: WO 2013/021093

(57) **Abstract**

The present invention relates to the use of cardiotrophin-1 (CT-1) for the prevention and/or treatment of acute renal injury, specially of acute kidney injury induced by nephrotoxic agents, such as contrast agents, antibiotics, immunosuppressive agents or antineoplastic agents. The invention is also related to compositions comprising said nephrotoxic agents and cardiotrophin-1.

## Description

### TECHNICAL FIELD OF THE INVENTION

The present invention relates to the use of cardiotrophin-1 (CT-1) for the prevention and/or treatment of acute kidney injury, especially of acute kidney injury induced by nephrotoxic agents. Likewise, the invention is also related to compositions comprising said nephrotoxic agents and cardiotrophin-1.

### BACKGROUND OF THE INVENTION

Acute kidney injury (AKI) is a complex process of damage in various kidney structures which leads to a sudden loss of complete or partial kidney function, producing acute kidney failure (AKF) in a short period of time which ranges from a few hours to a few days (López-Novoa JM. Kidney Int. 1999; 55:1672-1682; López-Novoa JM, et al. Kidney Int. 2011; 79:33-45). In general, the term AKI is used to define the reversible nature of most kidney aggressions; whilst the term AKF is reserved to describe the condition of those patients with a kidney injury that involves a decrease in glomerular filtrate and in the excretory capacity of the kidney, for which reason kidney replacement therapy (KRT) is required i.e. any intermittent or conventional dialysis method.

There are many mechanisms involved in the aetiology of AKI, among which the most important or best studied are (Schrier RW, et al. J Clin Invest. 2004;114:5-14): endothelial damage produced by vascular disturbances; tubular cell necrosis/apoptosis due to ischemia or due to the direct tubular effect of nephrotoxins; abolition of kidney regulation; inflammation; and necrosis and apoptosis of the tubular cells which leads to tubular obstruction. On many occasions, these mechanisms are inter-associated (López-Novoa JM, et al. Kidney Int. 2011;79:33-45).

The action of nephrotoxic agents on the kidney is one of the most common causes of acute kidney injury. Among the nephrotoxic agents of most typical pharmacological origin we have contrast agents, antineoplastic agents and antibiotics.

The incidence of deterioration in kidney function induced by contrast agents has significantly increased in recent years as a consequence of the rising number of diagnostic and therapeutic intervention processes performed on patients, and also the increase in patients with risk situations, such as diabetes (Calvin et al, Nature Reviews Nephrology 2010; 6: 679-688) and advanced age (Laville and Juillard, J Nephrol. 2010;23:387-398). Nephropathydue to contrast agents, although usually reversible, is far from being a benign complication, since it entails a prolongation of the hospital stay and in some cases, in particular in high-risk patients, leads to irreversible deterioration of kidney function (Laville and Juillard, J Nephrol. 2010;23:387-398).

The treatment of contrast agent-induced AKI, once established, is limited to support care and dialysis. To date, multiple molecules have been assayed for its prevention, such as atrial natriuretic peptide, statins, prostaglandin/prostacyclin analogues, N-acetylcysteine or isotonic intravenous bicarbonate, but these preventive therapies have not managed to demonstrate a conclusive efficacy (Weisbord SD, Palevsky PM. Curr Opin Nephrol Hypertens. 2010; 19(6):539-549).

Antineoplastic agents are another cause of acute kidney injury in patients that suffer from cancer subjected to chemotherapy. Specifically, cisplatin is one of the most effective antineoplastic agents in the treatment of solid tumours, with a wide spectrum of action. However, dose-dependent accumulative nephrotoxicity is the main therapeutic limitation of this drug, requiring in some cases a reduction in the dose or discontinuity of the treatment. In general, the strategies to prevent kidney injury produced by the administration of cisplatin are based on saline infusions to induce diuresis of the solute and on an optimum adjustment of the antineoplastic dose. Although it has been discovered that amifostine is effective in the prevention of kidney failure, cases of Stevens-Johnson syndrome and toxic epidermal necrolysis have been described in patients treated with said drug (Darmon M. et al. Critical care. 2006; 10:211).

Furthermore, antibiotics such as gentamicin and, in general, aminoglycoside antibiotics are active principles widely used for the treatment of a large variety of infections. Their main drawback, and the main limitation of their use, is their nephrotoxicity, since despite a correct monitoring and hydration of the patients, acute kidney injury appears in 10-20% of treatments. There are currently no drugs or therapeutic strategies that make it possible to prevent or minimize the kidney injury caused by gentamicin, nor improve the recovery of the organ after withdrawal of the treatment.

International patent application WO 2006/134601 A2 discloses methods for the prevention or treatment of acute renal failure induced by HgCl₂ comprising the administration of a pharmaceutical composition containing as active agent a fusion protein formed by a member of the IL-6 family linked to a soluble receptor of said member. However, the administration of the isolated IL-6 polypeptide, in absence of its receptor, is not capable of preventing or treating the kidney injury.

United States patent US 7,022,666 B1 discloses methods for inducing the formation of kidney epithelium from mesenchymal precursors in subjects that suffer kidney failure comprising the administration of a ligand of the gp130 receptor in the presence of a metanephric mesenchymal growth factor. Said metanephric mesenchymal growth factor is essential for induction of the epithelium.

Therefore, there is a need in the state of the art to provide agents useful for the treatment and/or prevention of acute kidney injury, and in particular acute kidney injury caused by nephrotoxic agents.

### SUMMARY OF THE INVENTION

In a first aspect, the invention relates to a compound which induces cardiotrophin-1 activity (CT-1) for use in the prevention and/or treatment of acute kidney injury, wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

In another aspect, the invention relates to a composition comprising, together or separately, a compound which induces cardiotrophin-1 activity and a nephrotoxic agent.

In another aspect, the invention relates to a composition comprising a compound which induces cardiotrophin-1 activity and a contrast agent for use as a diagnostic agent.

Even in another aspect, the invention relates to a composition comprising a compound which induces cardiotrophin-1 activity and an antibiotic for use in the prevention and/or treatment of antibiotic-associated nephrotoxicity.

In another aspect, the invention relates to a composition comprising a compound which induces cardiotrophin-1 activity and an immunosuppressive agent for use in the prevention and/or treatment of immunosuppressive agent-associated nephrotoxicity.

In a last aspect, the invention relates to a composition comprising a compound which induces cardiotrophin-1 activity and an antineoplastic agent for use in the prevention and/or treatment of antineoplastic agent-associated nephrotoxicity.

### BRIEF DESCRIPTION OF THE FIGURES

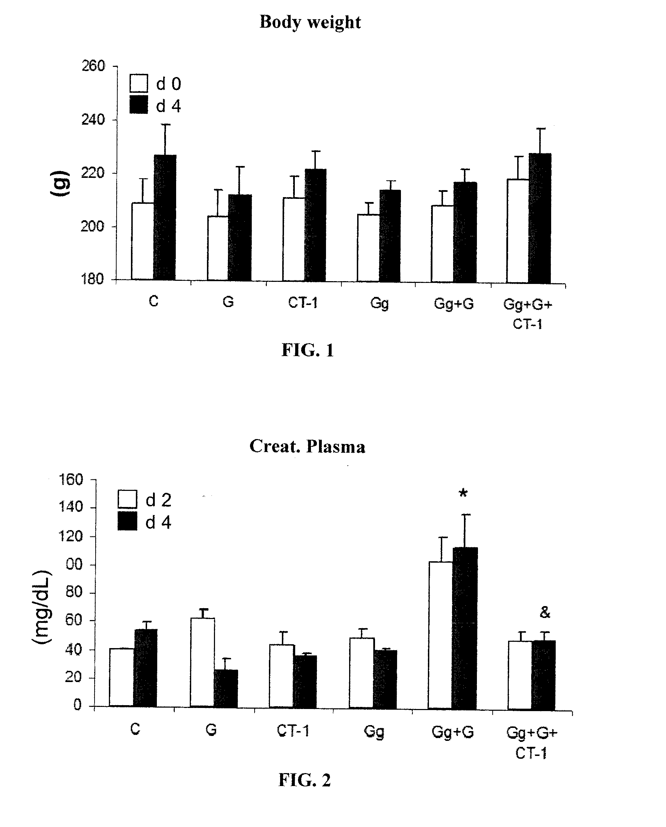

**Figure 1****.** Evolution of the body weight expressed in grams (g), during the experimental period in control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1). The data represent the mean ± SEM (4-5 animals/group). d 0: basal conditions on day 0; d 4: day 4 after the contrast.
**Figure 2****.** Creatinine plasma concentration (Creat. Plasma), expressed in mg/dL, of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1). Data on days 2 (d 2) and 4 (d 4) after administration of the gastrografin. The data represent the mean ± SEM of 4-5 animals/group. * p<0.05 with respect to the group C, G, CT-1 and Gg at the end of the treatment (fourth day after administration of the iodinated contrast). & p<0.05 with respect to the Gg+G at the end of the treatment. d 2: day 2 after the contrast; d 4: day 4 after the contrast.

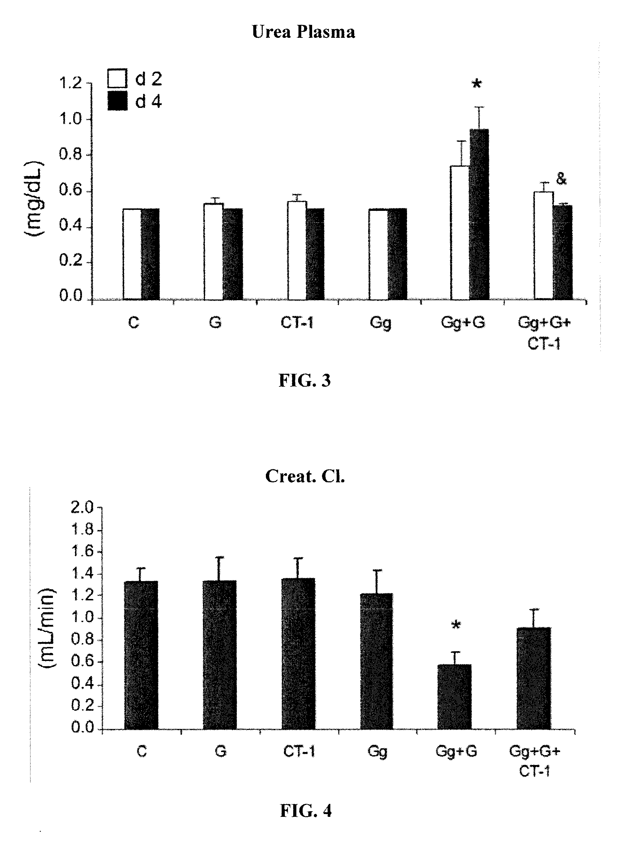

**Figure 3****.** Urea plasma concentration, expressed in mg/dL, of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1). Data from days 2 (d 2) and 4 (d 4) after administration of the gastrografin. The data represent the mean ± SEM of 4-5 animals.* p<0.05 with respect to the group C, G, CT-1 and Gg at the end of the treatment (fourth day after administration of the iodinated contrast). & p<0.05 with respect to the Gg+G at the end of the treatment. d 2:day 2 after the contrast; d 4: day 4 after the contrast.
**Figure 4****.** Creatinine clearance (Creat. Cl), expressed in ml/min, of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1), at the end of the treatments. The data represent the mean ± SEM of 4-5 animals/group. * p<0.05 with respect to the group C, G, CT-1.

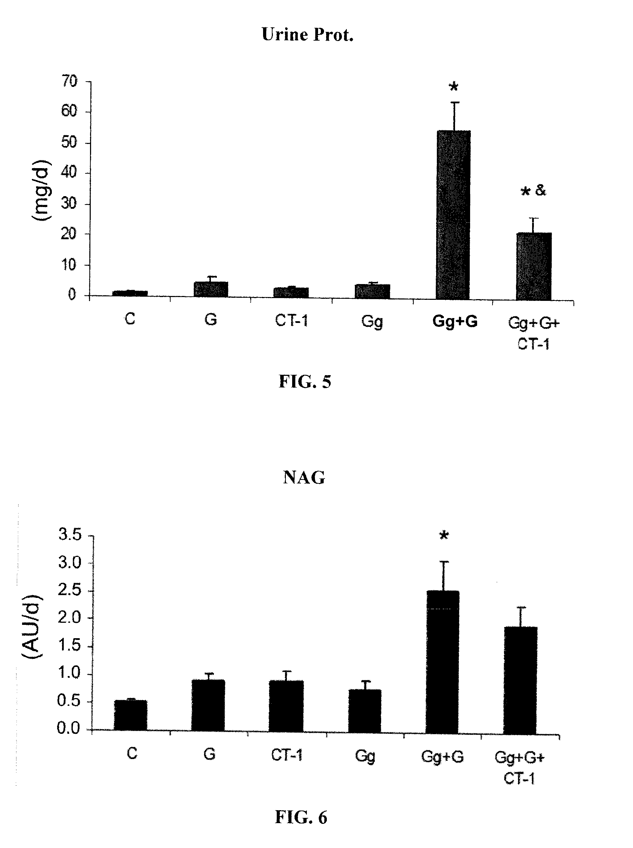

**Figure 5****.** Urinary excretion of proteins (Urine Prot.), expressed in mg/day (mg/d), of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1), at the end of the treatments. The data represent the mean ± SEM of 4-5 animals/group.* p<0.05 with respect to the group C, G, CT-1 and Gg. & p<0.05 with respect to the group Gg+G.
**Figure 6****.** Urinary excretion of N-acetyl glucosaminidase (NAG), expressed in arbitrary units per day (AU/d), of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1), at the end of the treatments. The data represent the mean ± SEM of 4-5 animals/group.* p<0.05 with respect to the group C, G, CT-1 and Gg.

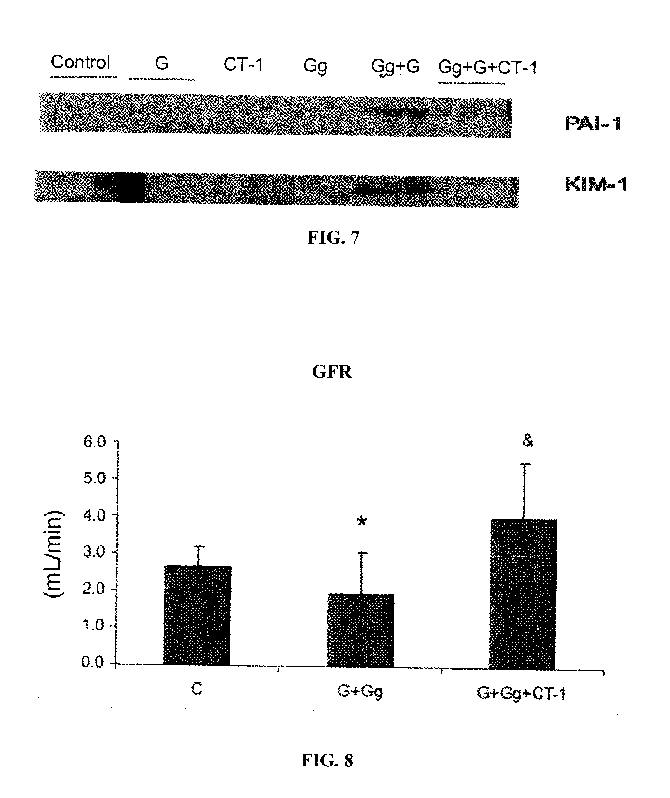

**Figure 7****.** Images representing Western blot analysis of KIM-1 and PAI-1 in the urine of control rats (C) and treated with gentamicin (G), cardiotrophin-1 (CT-1), gastrografin (Gg), gastrografin + gentamicin (Gg+G) and gastrografin + gentamicin + cardiotrophin-1 (Gg+G+CT-1), at the end of the treatments. Western Blot representing 3 experiments carried out separately.
**Figure 8****.** Glomerular filtration rate (GFR), expressed in mL/min of control rats (C) and treated with gastrografin + gentamicin (Gg+G) and gastrografin + gentamicin + cardiotrophin-1 (Gg+G+CT-1), at the end of the treatments. The data represent the mean ± SEM of 4 animals/group.* p<0.05 with respect to the group C. & p<0.05 with respect to the group Gg+G.

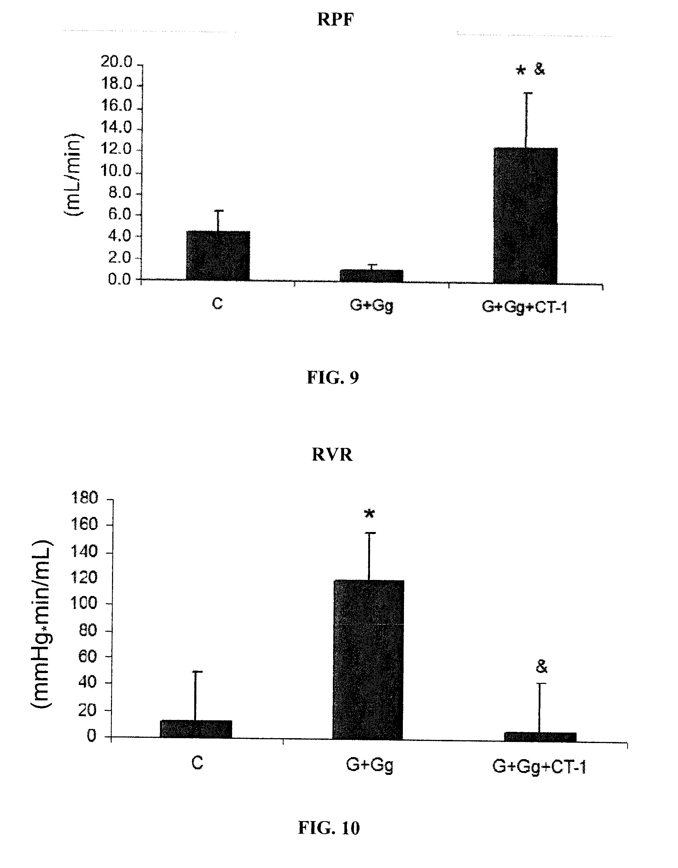

**Figure 9****.** Renal plasma flow (RPF), expressed in mL/min, of control rats (C) and treated with gastrografin + gentamicin (Gg+G) and with gastrografin + gentamicin + cardiotrophin-1 (Gg+G+CT-1), at the end of the treatments. The data represent the mean ± SEM of 4 animals/group.* p<0.05 with respect to the group C. & p<0.05 with respect to the group Gg+ G.
**Figure 10****.** Renal vascular resistance (RVR), expressed in mmHg·min/mL, of control rats (C) and treated with gastrografin + gentamicin (Gg+G) and gastrografin + gentamicin + cardiotrophin-1 (Gg+G+CT-1), at the end of the treatments. The data represent the mean ± SEM of 4 animals/group.* p<0.05 with respect to the group C. & p<0.05 with respect to the group Gg+G.

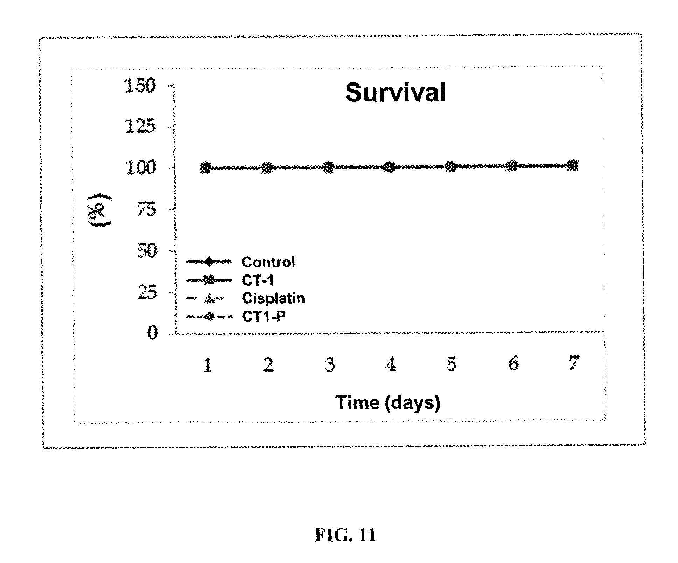

**Figure 11****.** Temporal evolution of the survival in the groups: vehicle (Control); cardiotrophin-1 (CT-1); cisplatin (Cisplatin) or cardiotrophin-1 and cisplatin (CT1-P). The data represent the mean ± SEM (n = 6 rats per group).

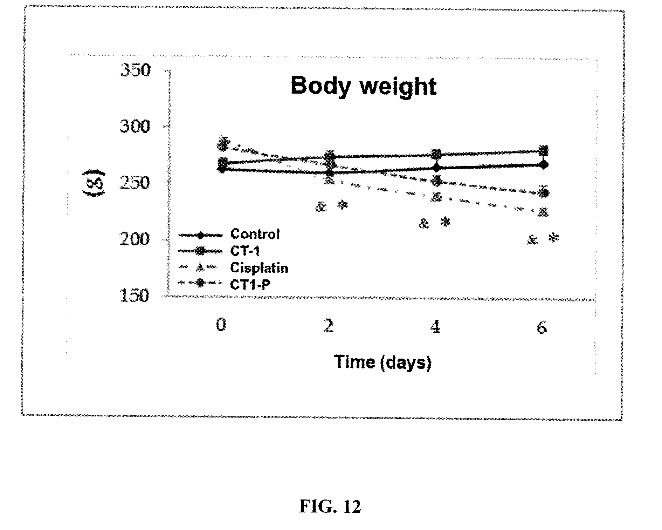

**Figure 12****.** Temporal evolution of body weight (grams, g) in the groups: vehicle (Control); cardiotrophin-1 (CT-1); cisplatin (Cisplatin) or cardiotrophin-1 and cisplatin (CT1-P). The data represent the mean ± SEM (n = 6 rats per group). * p<0.05 vs Control group, & p<0.05 vs Group Cardiotrophin-1-Cisplatin.

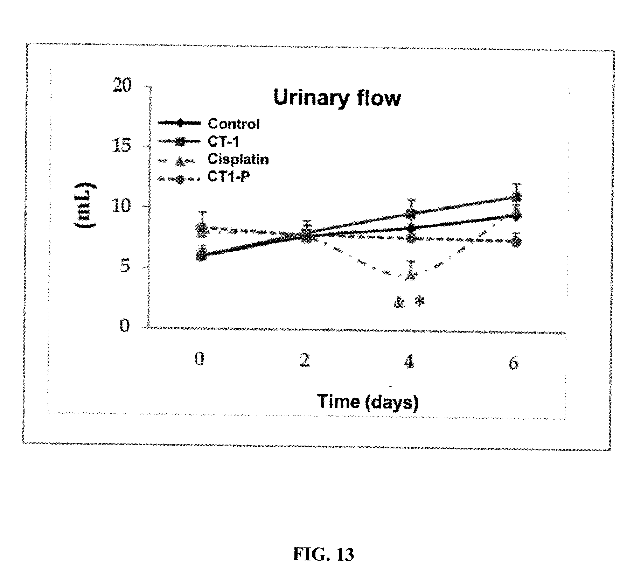

**Figure 13****.** Temporal evolution of the urinary flow (ml) in the groups: vehicle (Control); cardiotrophin-1 (CT-1); cisplatin (Cisplatin) or cardiotrophin-1 and cisplatin (CT1-P). The data represent the mean ± SEM (n = 6 rats per group). * p<0.05 vs Control group, & p<0.05 vs Group Cardiotrophin-1-Cisplatin.

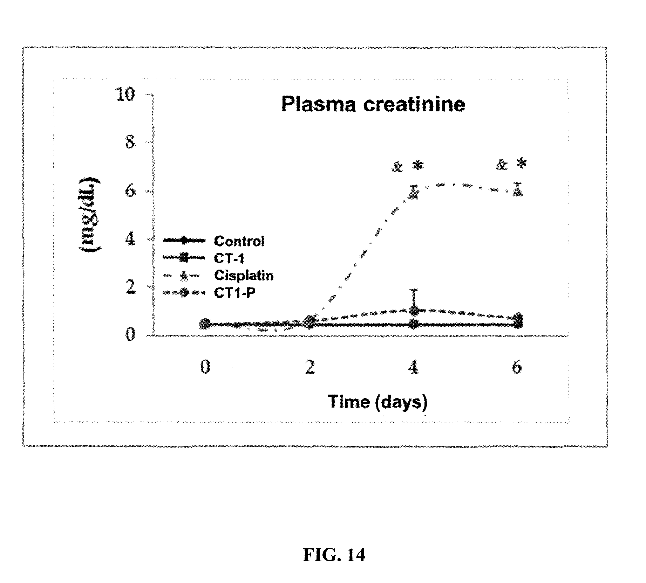

**Figure 14****.** Temporal evolution of the concentration of plasma creatinine (mg/dl) in the groups: vehicle (Control); cardiotrophin-1 (CT-1); cisplatin (Cisplatin) or cardiotrophin-1 and cisplatin (CT1-P). The data represent the mean ± SEM (n = 6 rats per group). * p<0.05 vs Control group, & p<0.05 vs Group Cardiotrophin-1-Cisplatin.

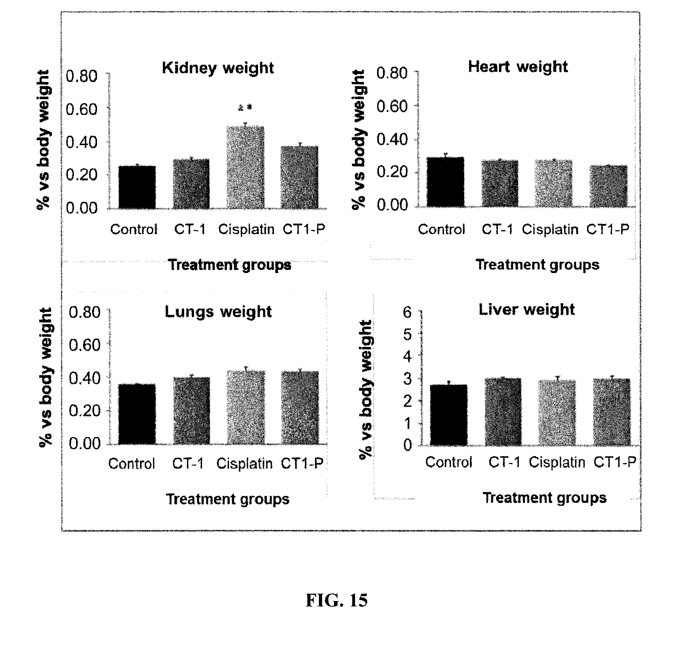

**Figure 15****.** Weight of the organs (with respect to the body weight, % vs body weight) at the end of the experimental period of the study in the groups: vehicle (Control); cardiotrophin-1 (CT-1); cisplatin (Cisplatin) or cardiotrophin-1 and cisplatin (CT1-P). The data represent the mean ± SEM (n = 6 rats per group). * p<0.05 vs Control group, & p<0.05 vs Group Cardiotrophin-1-Cisplatin.

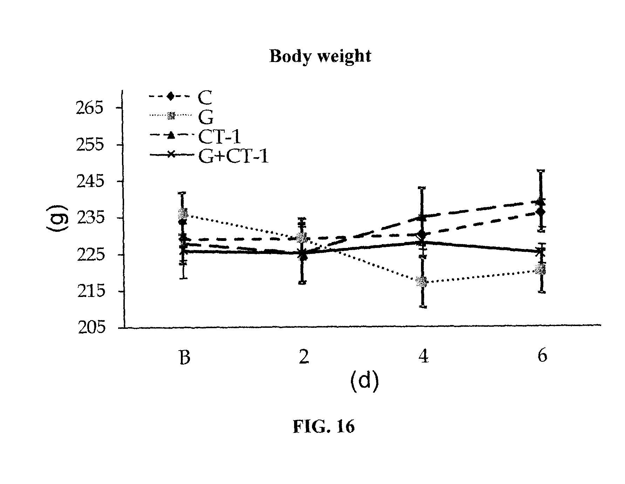

**Figure 16****.** Evolution of the body weight, expressed in grams (g), during the experimental period (d, days) in control rats (C) and treated with gentamicin (G), cardiotrophin-1 (CT-1) or both compounds (G + CT-1). The data represent the mean ± standard deviation of 5-6 animals. B: basal.

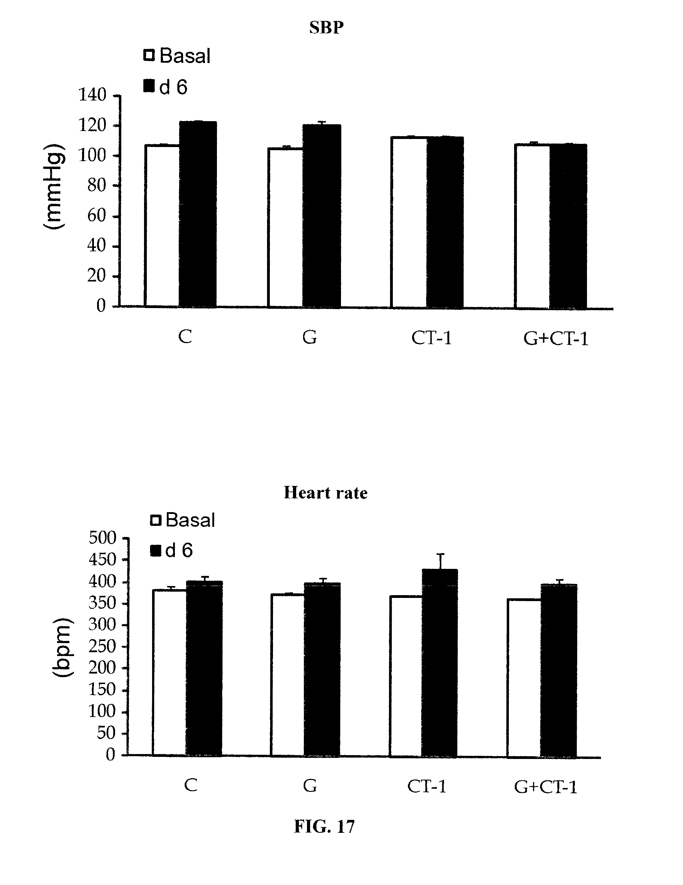

**Figure 17****.** Systolic blood pressure (SBP), upper panel, expressed in mmHg and heart rate (lower panel), expressed in bpm (beats per minute) of control rats (C) and treated with gentamicin (G), cardiotrophin-1 (CT-1) or both compounds (G + CT-1), before starting (Basal) and at the end of the treatments (d 6). The data represent the mean ± standard deviation of 5-6 animals.

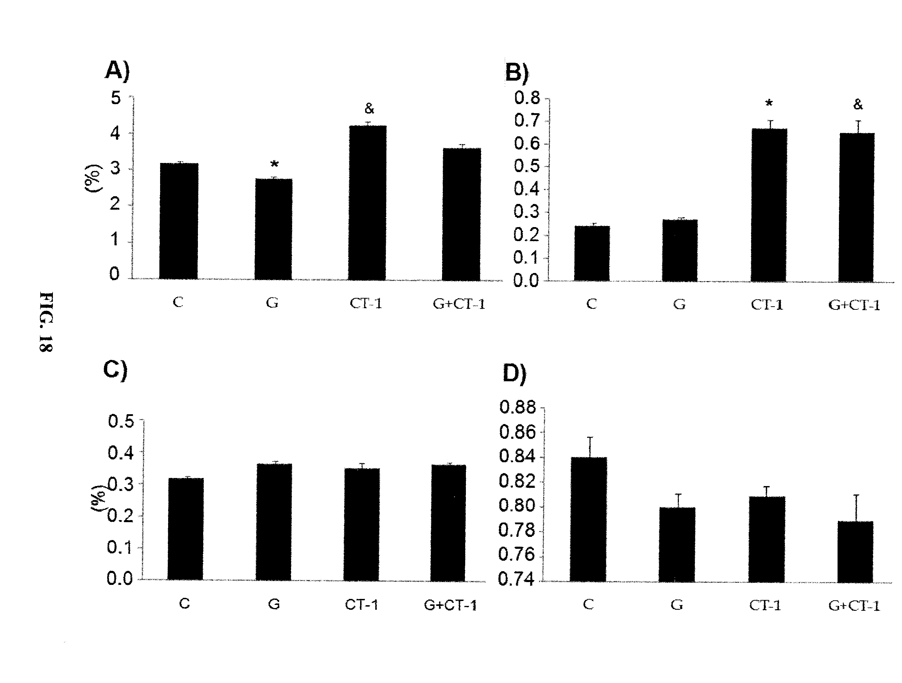

**Figure 18****.** Weight (% with respect to the body weight) of the liver (A), spleen (B), right kidney (C) and heart (D) of control rats (C) and treated with gentamicin (G), cardiotrophin-1 (CT-1) or both compounds (G + CT-1), at the end of the treatments. The data represent the mean ± standard deviation of 5-6 animals. * p<0.05 vs Control group, & p<0.05 vs Group G.

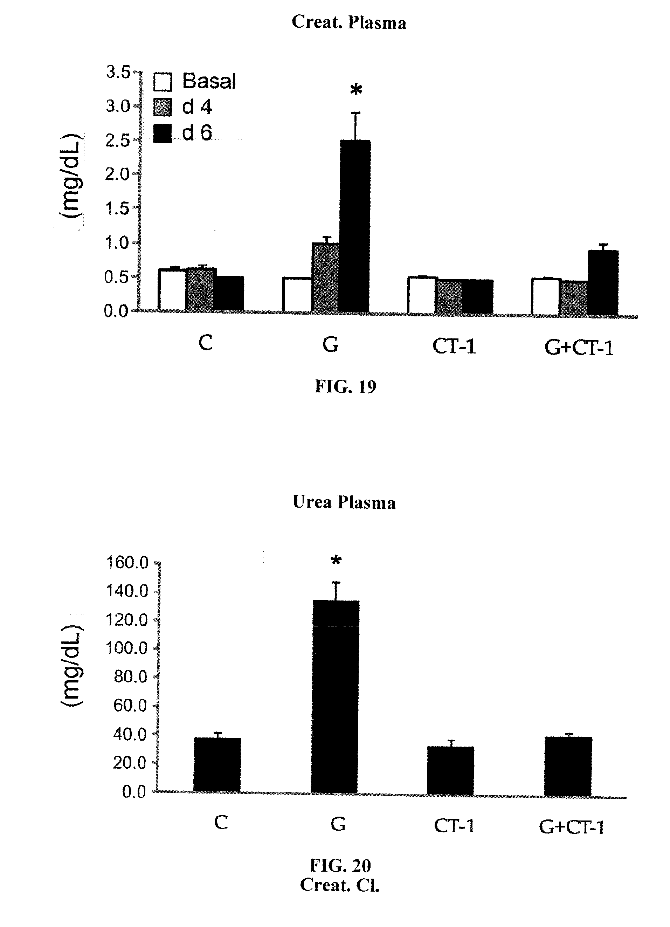

**Figure 19****.** Creatinine plasma concentration (Creat. Plasma), expressed in mg/dL, of control rats (C) and treated with gentamicin (G), cardiotrophin-1 (CT-1) or both compounds (G + CT-1), at the start of the experiment (Basal), after 4 days (d 4) and at the end of the study (d 6). The data represent the mean ± standard deviation of 5-6 animals. * p<0.05 vs Control group.
**Figure 20****.** Urea plasma concentration (Urea plasma) expressed in mg/dL, of control rats (C) and treated with gentamicin (G), cardiotrophin-1 (CT-1) or both compounds (G + CT-1), at the end of the treatments. The data represent the mean ± standard deviation of 5-6 animals. * p<0.05 vs Control group.

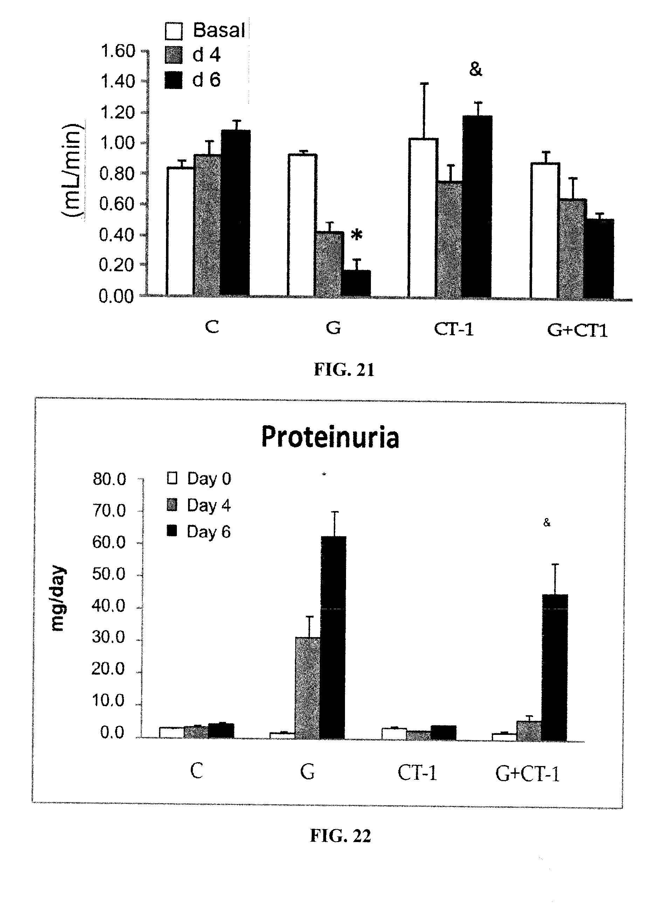

**Figure 21****.** Creatinine clearance (Creat. Cl.), expressed in mL/min, of control rats (C) and treated with gentamicin (G), cardiotrophin-1 (CT-1) or both compounds (G + CT-1), at the start of the study (Basal), after 4 days (d 4) and at the end of the study (d 6). The data represent the mean ± standard deviation of 5-6 animals. * p<0.05 vs Control group, & p<0.05 vs Group G.
**Figure 22****.** Urinary excretion of proteins (Proteinuria) expressed in mg/day of control rats (C) and treated with gentamicin (G), cardiotrophin-1 (CT-1) or both compounds (G + CT-1). The data represent the mean ± standard deviation of 5-6 animals. *p<0.05 vs Control group, & p<0.05 vs Group G.

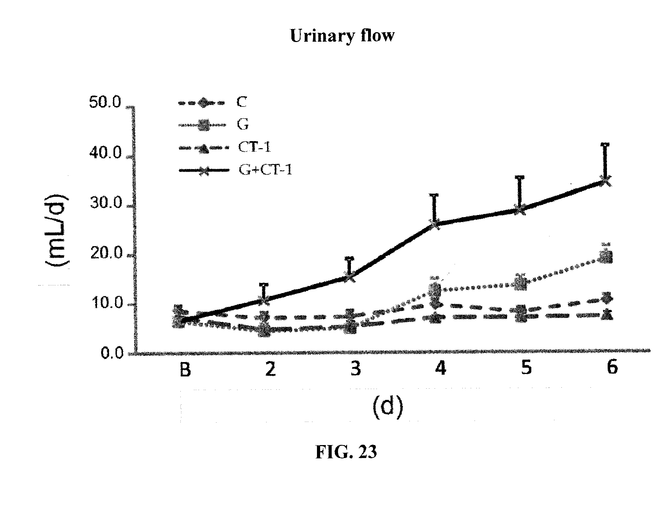

**Figure 23****.** Temporal evolution throughout the study (d, days) of the urinary flow expressed in mL/day (mL/d) of control rats (C) and treated with gentamicin (G), cardiotrophin-1 (CT-1) or both compounds (G + CT-1). The data represent the mean ± standard deviation of 5-6 animals.

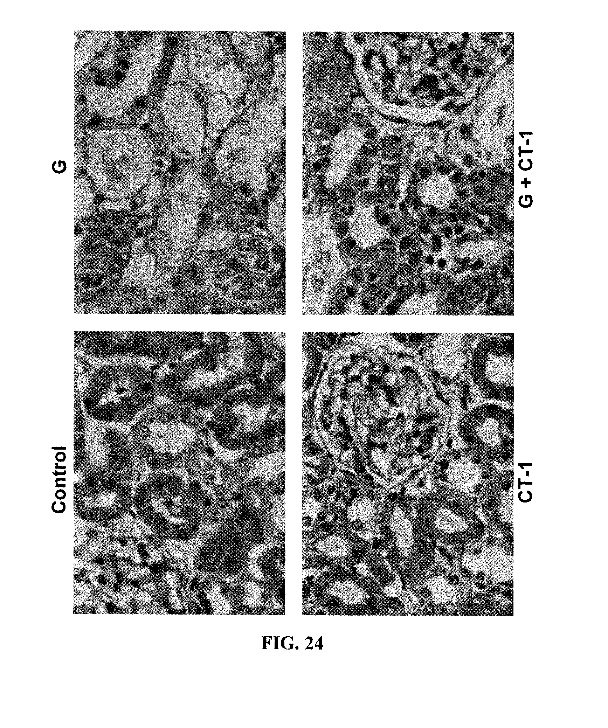

**Figure 24****.** Images representing optical microscopy (1,000 magnifications) of the cortical area of renal sections stained with hematoxylin and eosin, from control rats and treated with gentamicin (G), cardiotrophin-1 (CT-1) or both compounds (G+ CT-1), at the end of the treatments.

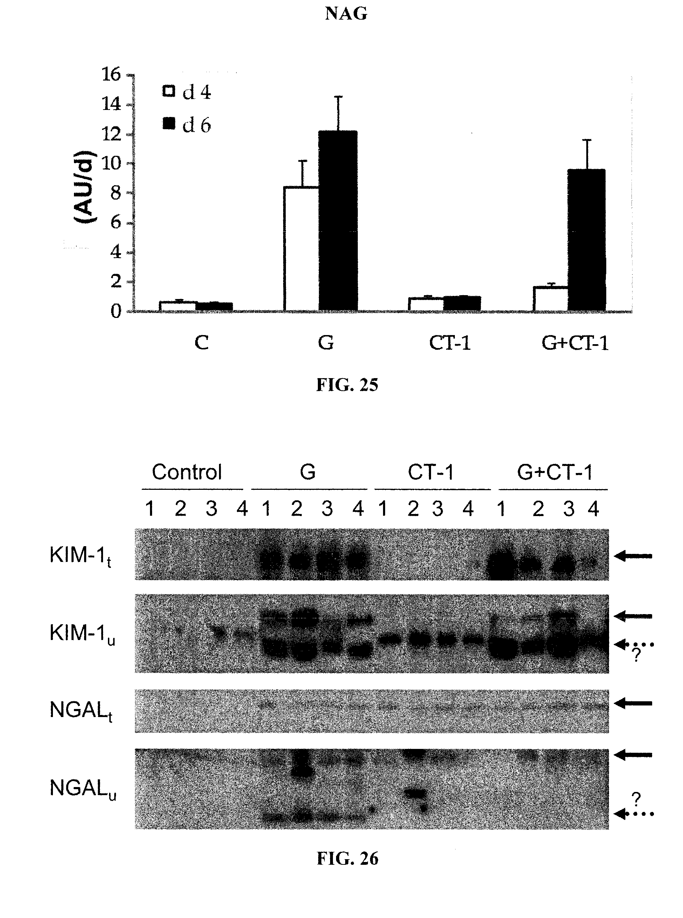

**Figure 25****.** Temporal evolution of the urinary excretion of N-acetyl glucosaminidase (NAG), expressed in arbitrary units per day (AU/d), of control rats (C) and treated with gentamicin (G), cardiotrophin-1 (CT-1) or both compounds (G + CT-1), after 4 days (d 4) after treatment and at the end of the study (d 6). The data represent the mean ± standard deviation of 5-6 animals.
**Figure 26****.** Images representing Western blot analysis of KIM-1 and NGAL in homogenates of renal tissue (t) and urines (u) of control rats and treated with gentamicin (G), cardiotrophin-1 (CT-1) or both compounds (G + CT-1), at the end of the treatments. The unbroken arrows indicate the bands obtained at the height of the expected molecular weights. The dotted arrows (and marked with a question mark symbol) indicate reactive bands in molecular weights less than that expected, which could correspond to fragments of the protein.

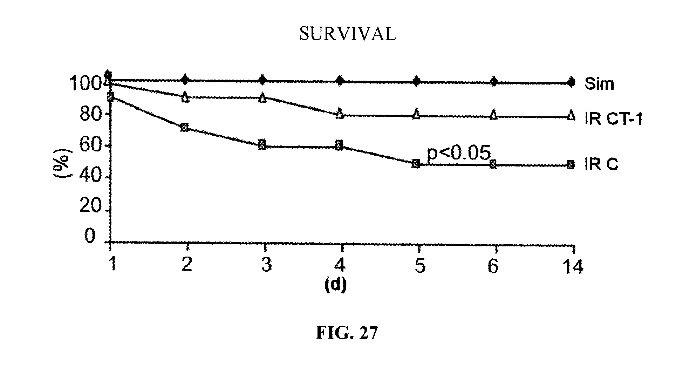

**Figure 27****.** Effect of the therapy with CT-1 in the survival of rats, expressed in percentage % of rats that survive, subjected to 60 minutes of left renal ischemia and 14 days (d) of reperfusion. Simulated rats (Sim), rats that received saline serum (IR C) and rats treated with CT-1 (IR CT-1) are represented.

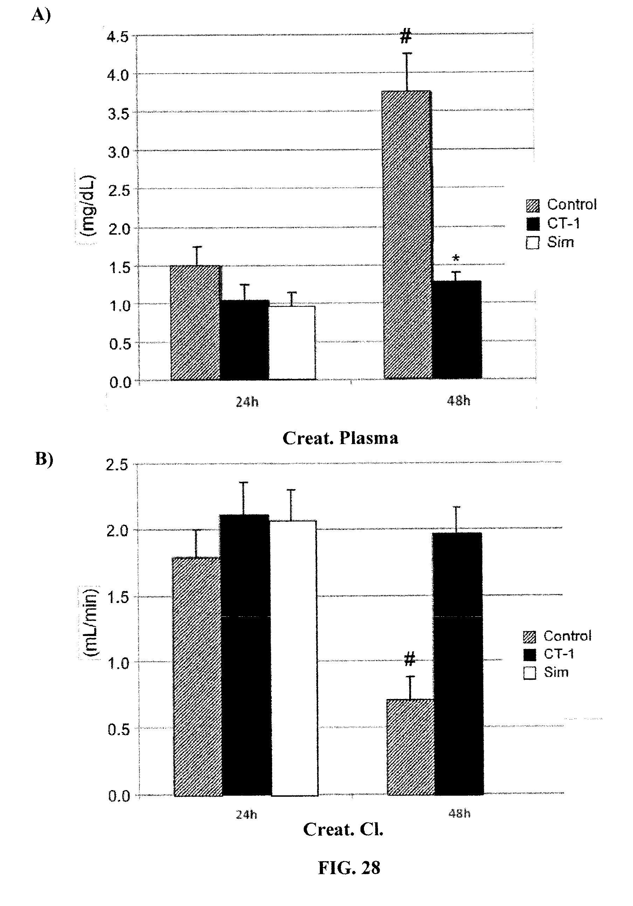

**Figure 28****.** Effect of the administration of CT-1: (A) on the creatinine plasma levels (Creat. Plasma), expressed as mg/dL, and (B) on the values of creatinine clearance (Creat. Cl.), expressed as mL/min, in animals subjected to left renal ischemia 24 and 48 hours post-reperfusion. Simulated rats (Sim), rats subjected to left renal ischemia that received saline serum (control) and rats subjected to left renal ischemia treated with CT-1 (CT-1) are represented. The data are represented as the mean ± SD of 5 rats per group. (*) P<0.05 vs. IR C; (#) P<0.05 vs. Sim.

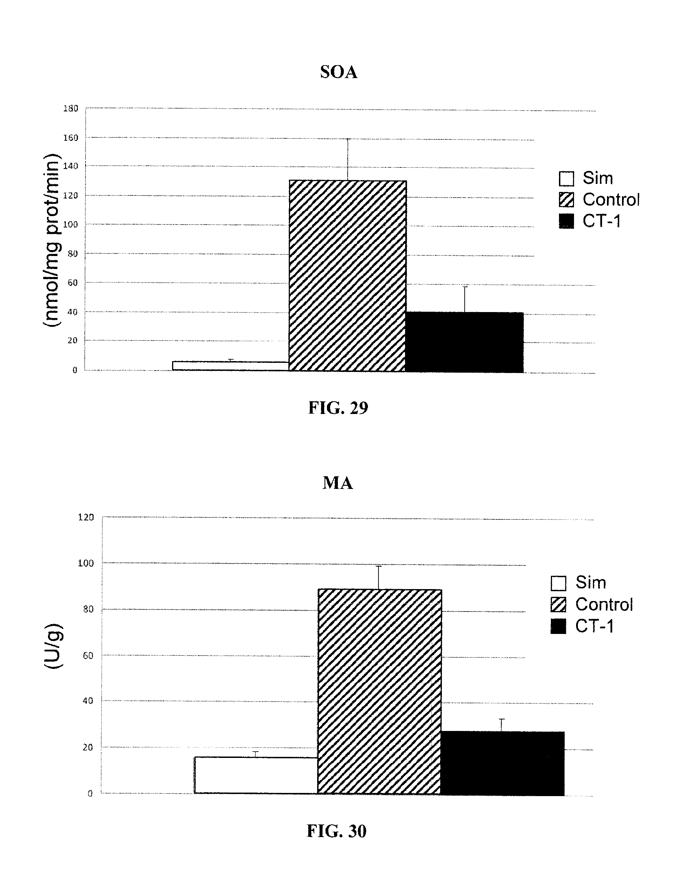

**Figure 29****.** Renal tissue levels of superoxide anion (SOA), expressed in nmol/mg of protein/min, in animals of the simulated group (Sim) and in two groups of animals subjected to left renal ischemia: ischemic control (control) and ischemic animals treated with CT-1 (CT-1) 4 hours after reperfusion. The data are expressed as the mean ± SD of 5 rats per group.
**Figure 30****.** Effect of the treatment with CT-1 on the myeloperoxidase activity (MA), expressed in units per gram of tissue (U/g), after renal I/R. The myeloperoxidase activity (MA) was measured 24 hours after the simulated operation or the reperfusion in renal tissue of animals of the simulated group (Sim) and in two groups of animals subjected to left renal ischemia: ischemic control (control) and animals treated with CT-1 (CT-1). The values are the mean ± SD of 5 rats per group.

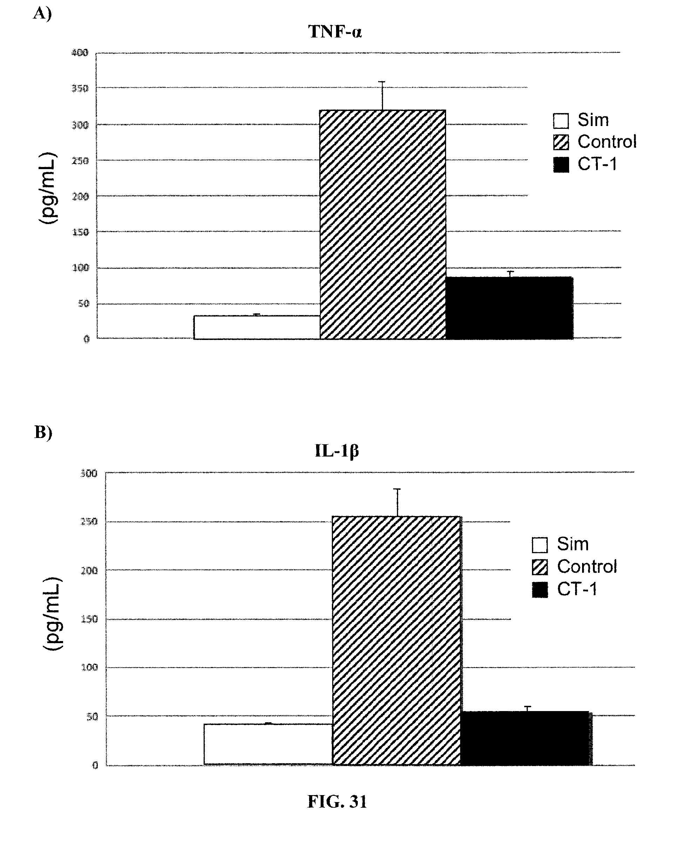

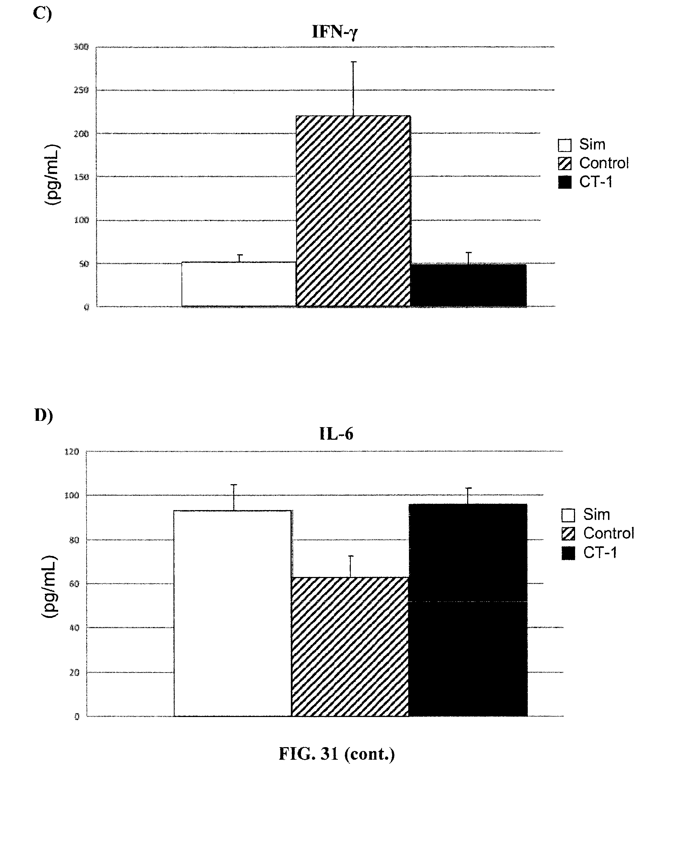

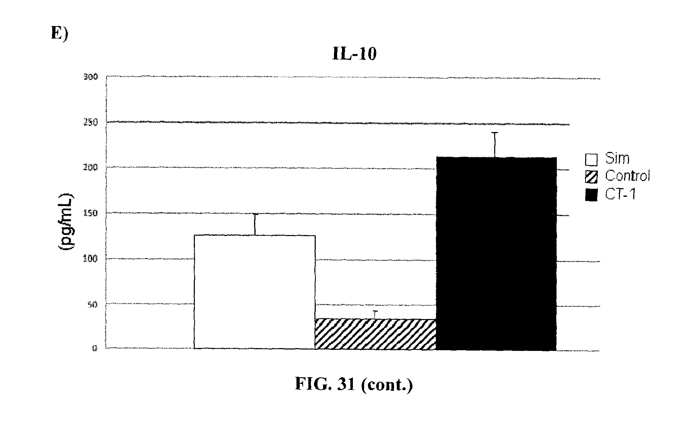

**Figure 31****.** Effect of the treatment with CT-1 on the plasma levels of pro-inflammatory cytokines, expressed in pg/mL, in animals of the simulated group (Sim) and in two groups of animals subjected to left renal ischemia: ischemic control (control) and ischemic animals treated with CT-1 (CT-1) 4 hours after reperfusion. **A)** Levels of TNF-α. **B)** Levels of IL-1β. **C)** Levels of IFN-γ. **D)** Levels of IL-6. **E)** Levels of IL-10. The data are expressed as the mean ± SD of 5 rats per group.

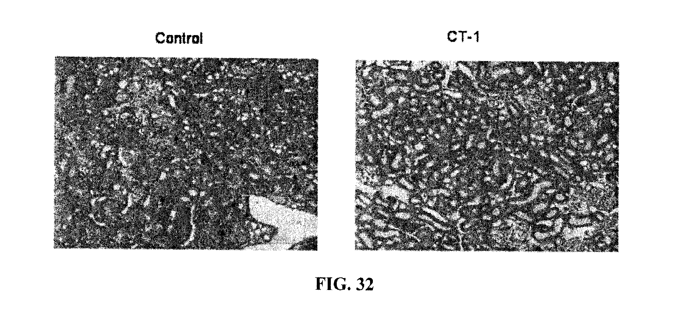

**Figure 32****.** Images representing hematoxylin-eosin staining of rat kidneys subjected to renal ischemia without treatment (control) and receiving cardiotrophin-1 (CT-1) 24 hours after reperfusion. The arrow tips indicate tubular cells with a lot of vacuolated cytoplasm. The arrows indicate obstructed tubules.

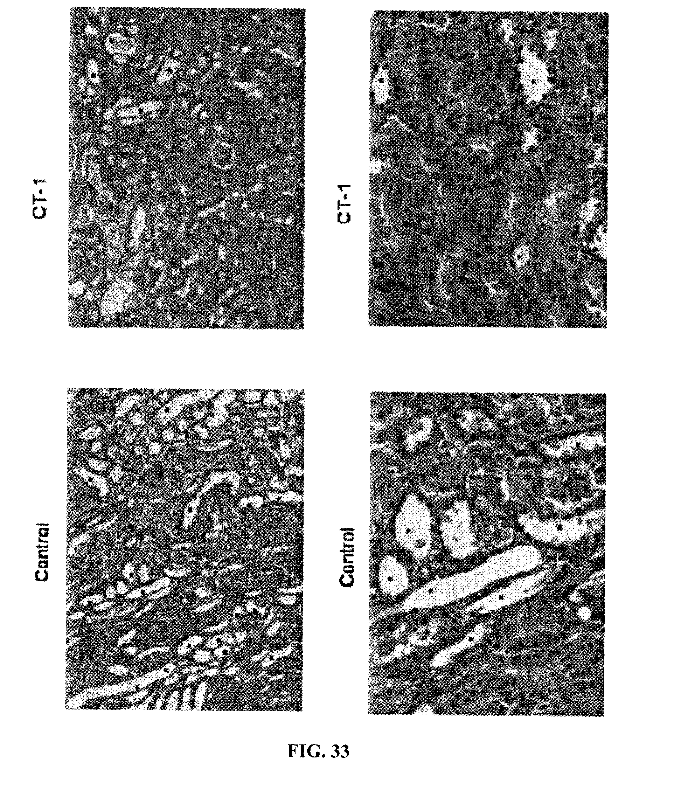

**Figure 33****.** Images representing hematoxylin-eosin staining of rat kidneys subjected to renal ischemia without treatment (control) and receiving cardiotrophin-1 (CT-1) 48 hours after reperfusion. The asterisks indicate completely denuded tubules. The arrow tips indicate areas with inflammatory infiltrate.

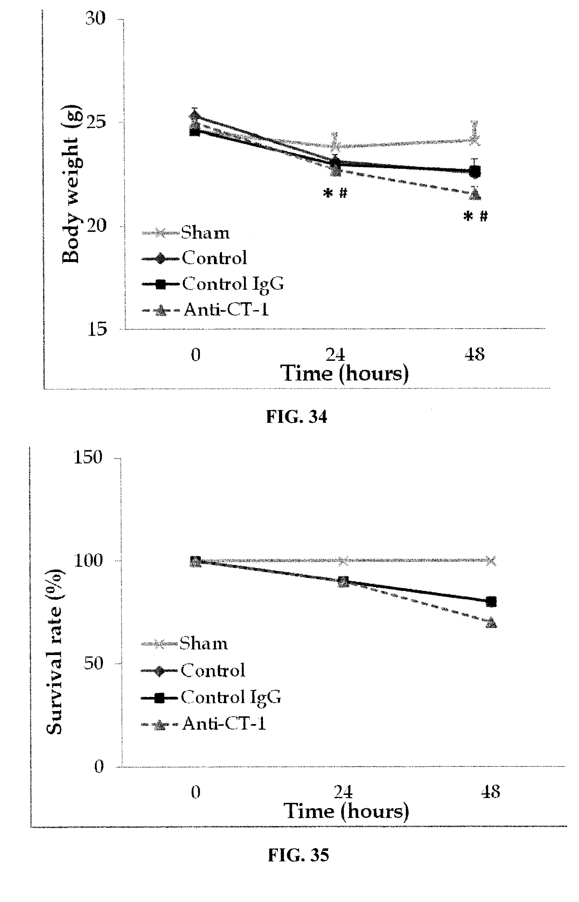

**Figure 34****.** Effect of anti-CT-1 antibody on the body weight, expressed in grams (g), in mice subjected to renal ischemia-reperfusion (I/R). Ischemia injury was induced by 30 minutes microvascular clamping of the left renal pedicle. Experimental groups are: Sham (n = 6), Control (n = 10), Control IgG (n = 10) and Anti-CT-1 (n = 10). Values are expressed as mean ± SEM of n independent experiments. Statistically significant differences: *, p < 0.05 in Anti-CT-1 group vs. time 0; #, p < 0.05 in Control IgG group vs. time 0.
**Figure 35****.** Effect of anti-CT-1 antibody on survival rate, expressed in percentage (%), in mice subjected to renal ischemia-reperfusion (I/R). Experimental groups are: Sham (n = 6), Control (n = 10), Control IgG (n = 10) and Anti-CT-1 (n = 10). Values are expressed as mean ± SEM of n independent experiments.

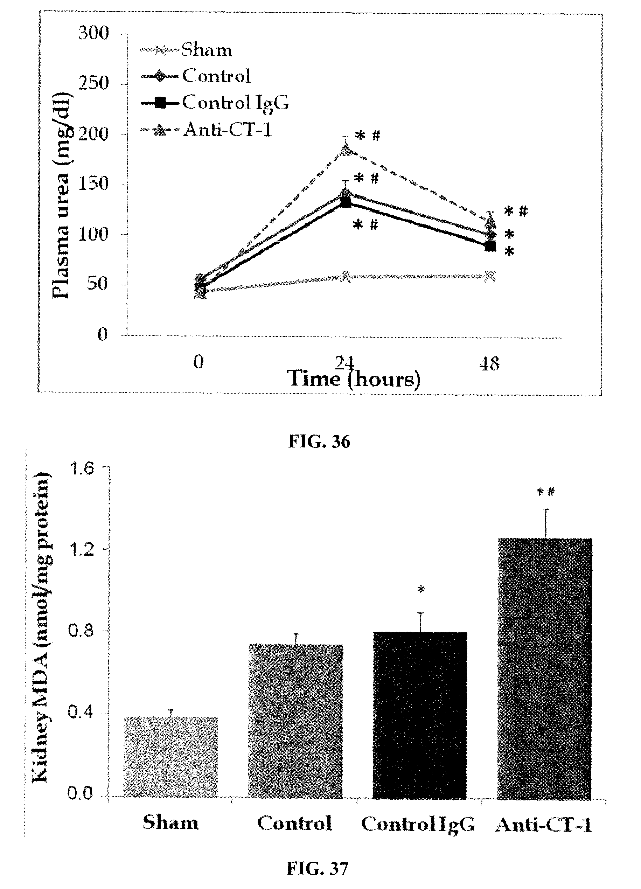

**Figure 36****.** Effect of anti-CT-1 antibody on plasma urea, expressed in mg/dl, in mice subjected to renal ischemia/reperfusion (I/R). Experimental groups are: Sham (n = 6), Control (n = 10), Control IgG (n = 10) and Anti-CT-1 (n = 10). Values are expressed as mean ± SEM of n independent experiments. Statistically significant differences, *, Z > 1.96, different times vs. time 0; by time, #, Z > 1.96, each group vs. Sham.
**Figure 37****.** Effect of anti-CT-1 antibody on kidney malondialdehyde (MDA) levels, expressed in nmol/mg protein, in mice subjected to renal isquemia-reperfusion (I/R). Values are expressed as mean ± SEM of n independent experiments. Experimental groups are: Sham (n = 6), Control (n = 10), Control IgG (n = 10) and Anti-CT-1 (n = 10), at 48 hours after surgical operation. Statistically significant differences, *, Z > 1.96, vs. Sham; #, Z > 1.96, vs. Control.

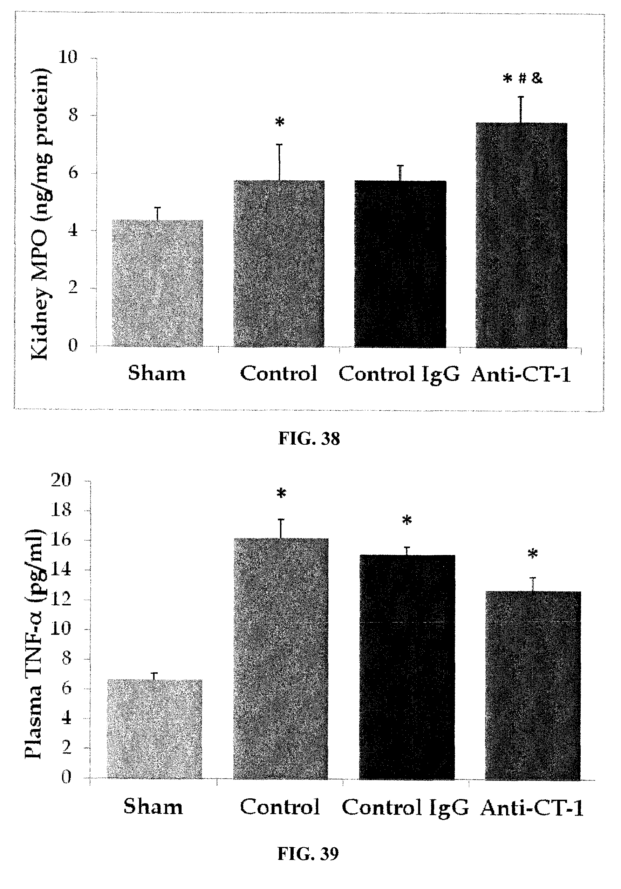

**Figure 38****.** Effect of anti-CT-1 antibody on kidney myeloperoxidase (MPO) levels, expressed in ng/mg protein, in mice subjected to renal ischemia-reperfusion (I/R). Values are expressed as mean ± SEM of n independent experiments. Experimental groups are: Sham (n = 6), Control (n = 10), Control IgG (n = 10) and Anti-CT-1 (n = 10), at 48 hours after surgical operation. Statistically significant differences, by groups: *, Z > 1.96, vs. Sham; #, Z > 1.96, vs. Control; &, Z > 1.96, vs. Control IgG.
**Figure 39****.** Effect of anti-CT-1 antibody on plasma tumor necrosis factor-alpha (TNF-α) levels, expressed in pg/ml, in mice subjected to renal ischemia-reperfusion (I/R). Values are expressed as mean ± SEM of n independent experiments. Experimental groups are: Sham (n = 4), Control (n = 4), Control IgG (n =8) and Anti-CT-1 (n = 8), at 4 hours after surgical operation. Statistically significant differences, by groups: *, p < 0.05, each group, vs. Sham.

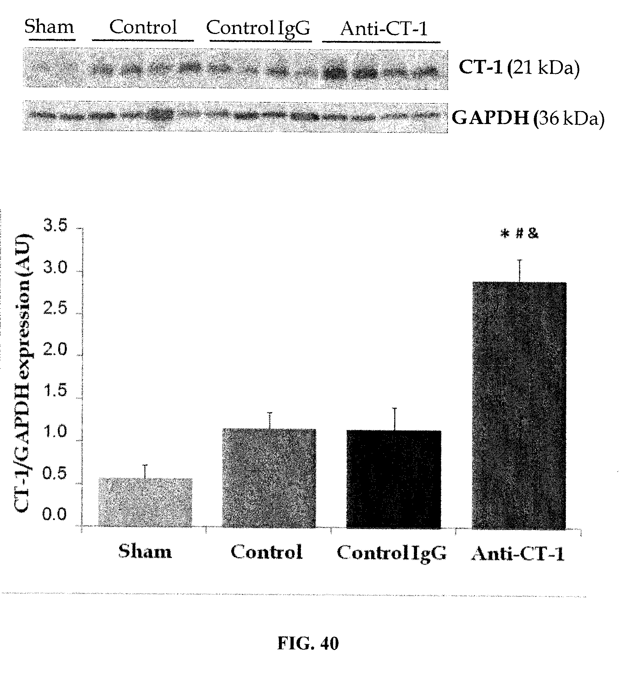

**Figure 40****.** Effect of anti-CT-1 antibody on kidney cardiotrophin-1 (CT-1) expression in mice subjected to renal ischemia-reperfusion (I/R). Densitometric analysis of kidney expression of cardiotrophin-1/glyceraldehyde-3-phosphate dehydrogenase (CT-1/GAPDH), expressed in arbitrary units (AU). Values are expressed as mean ± SEM of n independent experiments. Experimental groups are: Sham (n = 6), Control (n = 10), Control IgG (n = 10) and Anti-CT-1 (n = 10), at 48 hours after surgical operation. Statistically significant differences, by groups: *, p < 0.05, vs. Sham; #, p < 0.05, vs. Control; &, p < 0.05, vs. Control IgG.

### DETAILED DESCRIPTION OF THE INVENTION

The authors of the present invention have surprisingly observed that cardiotrophin-1 (CT-1) is useful for the prevention and/or treatment of acute kidney injury, especially of the acute kidney injury induced by a nephrotoxic agent or caused by ischemia/reperfusion. Said effect depends on the capacity of CT-1 of:
- reducing the deterioration of renal excretory function reducing the decrease of creatinine clearance (Figures 4, 21 and 28B); reducing the accumulation in the blood of metabolism products such as creatinine (Figures 2, 14, 19 and 28A), urea and their derivatives (Figures 3 and 20); and decreasing the appearance of proteinuria (Figures 5 and 22);
- reducing polyuria measured by the evolution of urinary flow (Figures 13 and 23);
- reducing nephrotoxicity, measured by the urinary markers of kidney damage: N-acetyl glucosaminidase (NAG) (Figures 6 and 25), *"Kidney Injury Molecule 1"* (KIM-1) (Figure 7) and plasminogen activator inhibitor 1 (PAI-1) (Figure 7);
- protecting from the vascular and glomerular effects involved in the nephrotoxicity avoiding the decrease in glomerular filtration rate (GFR) (Figure 8) and renal plasma flow (RPF) (Figure 9); and avoiding the increase in renal vascular resistance (RVR) (Figure 10);
- reducing the loss in body weight produced by a nephrotoxic agent (Figure 16);
- reducing renal tissue damage observed in histological studies (Figures 24, 32 and 33);
- lessening oxidative stress caused by ischemia-reperfusion, decreasing the production of oxygen free radicals (Figure 29);
- reducing the activation of inflammatory response, decreasing myeloperoxidase activity (MPO) (Figure 30), decreasing the production of pro-inflammatory cytokines (TNFα, IL-1β, IFN-γ) and avoiding the decrease in plasma levels ofIL-6 and IL-10 (Figure 31).

Another object of the present invention is to provide compositions comprising a nephrotoxic agent (such as a contrast agent, an antibiotic, an immunosuppressive agent or an antineoplastic agent) and cardiotrophin-1, so that the administration of cardiotrophin-1 together with the nephrotoxic agent makes it possible to improve the pharmacotoxicological profile thereof and, therefore, improving its use and application.

### THERAPEUTIC USES OF THE INVENTION

In a first aspect, the invention relates to a compound which induces cardiotrophin-1 activity (CT-1) for use in the prevention and/or treatment of acute kidney injury, wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

Additionally, the invention relates to the use of a compound which induces cardiotrophin-1 activity (CT1) for the preparation of a medicament for the prevention and/or treatment of acute kidney injury, wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

The invention is also related to a method of treatment of acute kidney injury comprising the administration to a subject of a compound which induces cardiotrophin-1 activity (CT-1), wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

The expression "compound which induces cardiotrophin-1 activity", as used in the present document, is understood to be any compound the administration whereof causes an increase in cardiotrophin-1 activity (CT-1) irrespective of the fact that said increase is caused by an increase in the specific activity of the pre-existing CT-1 or by an increase in CT-1 synthesis or of analogues thereof which substantially share the same function as cardiotrophin. Thus, in a preferred embodiment, the agent which induces CT-1 activity is CT-1 itself.

The term "cardiotrophin 1" or "CT-1", as used in the present invention, relates to a cytokine belonging to the interleukin 6 family, capable of linking and activating the signalling mediated at least by the complex of the LIFR receptor consisting of the heterodimer gp130/LIFRβ. In the present invention, "CT-1" is understood to be the protein defined by the sequence of the NCBI database with accession number NP_001321.1 dated 9 April 2011 which corresponds to the sequence SEQ ID NO:1, corresponding to isoform 1 of human cardiotrophin; or the protein defined by the sequence of the NCBI database with accession number NP_001136016.1 dated 12 March 2011, corresponding to isoform 2 of human cardiotrophin. In a preferred embodiment, the CT-1 is a CT-1 of human origin, preferably of sequence SEQ ID NO: 1.

SEQ ID NO: 1

MSRREGSLED PQTDSSVSLL PHLEAKIRQT HSLAHLLTKY AEQLLQEYVQ LQGDPFGLPS 60
FSPPRLPVAG LSAPAPSHAG LPVHERLRLD AAALAALPPL LDAVCRRQAE LNPRAPRLLR 120
RLEDAARQAR ALGAAVEALL AALGAANRGP RAEPPAATAS AASATGVFPA KVLGLRVCGL 180
YREWLSRTEG DLGQLLPGGS A 201

The invention contemplates the use of functionally equivalent variants of CT-1. "Functionally equivalent variant of CT-1", as used here, is understood as all molecules that share with CT-1 one or more of the functions described in the present invention associated to CT-1, both *in vitro* and *in vivo,* and which has a minimum identity in the amino acid sequence. In the present invention, the expression "functionally equivalent variant of CT-1" aims to exclude other ligands of gp130, such as leukaemia inhibitory factor (LIF), IL-11, IL-6, oncostatin M (OSM), ciliary neurotrophic factor (CNTF), or cardiotrophin-like cytokine (CLC). The "functionally equivalent variants of CT-1" of the present invention must have an identity with CT-1 of, at least, 60%.

Thus, in an embodiment, the compound which induces cardiotrophin-1 activity is CT-1 or a functionally equivalent variant thereof which has, at least, 60% identity with CT-1.

The variants of CT-1 may be both natural and artificial.

The expression "natural variant" relates to all those variants of human CT-1 mentioned above which appear naturally in other species, i.e. the orthologues of CT-1. Said natural variants include, without limitation, mouse CT-1, which corresponds to the sequence with accession number Q541U3 dated 28 November 2006 or to the sequence Q60753 dated 31 May 2011 in the NCBI database or to the isoform of 196 amino acids which corresponds to the sequence with accession number P83714 dated 31 May 2011 in the NCBI database; rat CT-1, which corresponds to the sequence with accession number Q63086 dated 8 March 2011; macaque CT-1, which corresponds to the sequence with accession number XP_001103315 dated 1 June 2010; dog CT-1, which corresponds to the sequence with accession number XP_849072 dated 30 August 2005; horse CT-1, which corresponds to the sequence with accession number XP_001915457 dated 11 July 2008; and CT-1 of bovine origin, which corresponds to the sequence with accession number NP_001179313 dated 14 November 2010. The natural variants of CT-1 suitable for their use in the present invention may also derive from said sequences by insertion, substitution or deletion of one or more amino acids and include natural alleles (such as variant A92T of human CT-1), variants resulting from alternative processing and secreted and truncated forms which appear naturally.

The CT-1 useful in the present invention may, therefore, be of natural sequence, when it comprises a polypeptide which has the same sequence of amino acids as the CT-1 which appears in nature. Said polypeptides of natural sequence may be isolated from nature or can be produced by recombinant and/or synthetic means. Thus, the CT-1 of the invention may be a recombinant protein obtained by the expression of a polynucleotide which codes for CT-1 or a functionally equivalent variant thereof in a heterologous organism, such as a bacterium, yeast or insect or mammalian cell. Said recombinant protein may be obtained as a fusion protein with an amino-terminal tail of histidines which facilitates its later purification. The expression and purification of said proteins can be performed according to methods known by persons skilled in the art and described in the state of the art.

In a preferred embodiment, the CT-1 is of human origin and corresponds to the sequence SEQ ID NO:1. In another preferred embodiment, the CT-1 is from a rat, preferably is a fusion protein with an amino-terminal tail of histidines, more preferably of SEQ ID NO:2.

SEQ ID NO:2

MRGSHHHHHH GMASMTGGQQ MGRDLYDDDD KDRWGSMSQR EGSLEDHQTD SSFSFLPHLE 60
AKIRQTHNLA RLLTKYADQL LEEYVQQQGE PFGLPGFSPP RLPLAGLSGP APSHAGLPVS 120
ERLRQDAAAL SALPALLDAV RRRQAELNPR APRLLRSLED AARQVRALGA AVETVLAALG 180
AAARGPVPEP VATSALFTSN SAAGVFSAKV LGLHVCGLYG EWVSRTEGDL GQLVPGGVA 239

Alternatively, the CT-1 may be a functionally equivalent artificial variant of CT-1, which can be obtained by recombinant and/or synthetic means.

The variants of CT-1 contemplated in the present invention show, at least, some of the functions of CT-1 such as, without limitation:
- the capacity to reduce serum creatinine levels and increasing creatinine clearance. A suitable method to determine these parameters is detailed in the Materials and methods section of the present invention.
- the capacity to reduce urea levels and its derivatives. A suitable method to determine these parameters is detailed in the Materials and methods section of the present invention.
- the capacity to decrease proteinuria. A suitable method to determine this parameter is detailed in the Materials and methods section of the present invention.
- the capacity to reduce polyuria, which can be determined by the evolution of the urinary flow, as detailed in the Materials and methods section of the present invention.
- the capacity to reduce the production of urinary markers of kidney injury, such as N-acetyl glucosaminidase (NAG) (determined as detailed in the Materials and methods section of the present invention), or the *"Kidney Injury Molecule 1"* (KIM-1) molecules and the plasminogen activator inhibitor 1 (PAI-1), which can be determined by Western blot, as shown in the Materials and methods section of the present invention.
- the capacity to increase glomerular filtration rate (GFR) and renal plasma flow (RPF) and to decrease renal vascular resistance (RVR), which can be determined by the methods described in the state of the art, such as that shown in the Materials and Methods section of the present invention.
- the capacity to reduce renal tissue damage, determined by histological studies of renal sections, which can be determined by the methods described in the state of the art, such as that shown in the Materials and Methods section of the present invention.
- the capacity to decrease the production of oxygen free radicals, which can be determined by methods described in the state of the art, such as that shown in the Materials and Methods section of the present invention.
- the capacity to prevent the activation of pro-inflammatory signalling pathways, determined by myeloperoxidase activity (MPO), which can be determined by the method detailed in the Material and methods section of the present invention.
- the capacity to decrease the production of pro-inflammatory cytokines (TNFα, IL-1β, IFN-γ) and to increase the plasma levels of IL-6 and IL-10, which can be determined by the methods detailed in the Materials and Methods section of the present invention.

Additionally, functionally equivalent variants of CT-1 contemplated in the context of the present invention, include polypeptides which show at least 60%, 70%, 80%, 85%, 90%, 92%, 94%, 96%, 98%, 99% of similarity or identity with the different natural variants of CT-1 mentioned above.

The percentage of identity between two sequences indicates the proportion of identical amino acids that share the two sequences that are compared, whilst the percentage of similarity indicates the proportion of residues of similar amino acids (considering equivalent the residues of amino acids such as arginine and lysine or aspartic acid and glutamic acid). The percentage of identity between two sequences of amino acids is calculated by comparing two sequences aligned on a particular region, determining the number of positions wherein there are identical amino acids in both sequences to obtain the number of coincident positions by dividing the number of said positions by the number of total positions in the segment which is being compared and multiplying the result by 100. The degree of identity and similarity between two polypeptides is determined using computer-implemented algorithms and methods that are widely known by persons skilled in the art. The identity and similarity between two sequences of amino acids is preferably determined using the BLASTP algorithm (BLAST Manual, Altschul, S. et al., NCBI NLM NIH Bethesda, Md. 20894, Altschul, S., et al., J., 1990, Mol. Biol. 215:403-410).

In another embodiment of the invention, the compound which induces cardiotrophin-1 activity is a polynucleotide which codes for CT-1 or a functionally equivalent variant of CT-1 which has, at least, 60% identity with CT-1. In an embodiment, the functionally equivalent variant of CT-1 has, at least, 60%, 70%, 80%, 85%, 90%, 92%, 94%, 96%, 98%, 99% of identity with CT-1.

The term "polynucleotide", as used in the present invention, relates to a polymeric form of nucleotides of any length and formed by ribonucleotides and/or deoxyribonucleotides. The term includes both single and double stranded polynucleotides, as well as modified polynucleotides (methylated, protected and similar).

Suitable polynucleotides for their use as agents capable of inducing CT-1 activity include, without limitation, the polynucleotides whose sequences correspond to those described in the GenEMBL database with accession numbers BC064416 in the version 9 dated 15.10.2008, corresponding to variant 1 of the transcript of human cardiotrophin, BC036787 in version 9 dated 12.08.2009, corresponding to variant 1 of the transcript of human cardiotrophin, the sequence described in the GenEMBL database with accession number D78591, in its version 4 dated 12.01.2009, corresponding to the polynucleotide which codes for cardiotrophin 1 of *Rattus norvegicus* (rat), the sequence described in the GenEMBL database with accession number AY518205, in its version 3 dated 12.01.2009, corresponding to the polynucleotide which codes for cardiotrophin 2 of *Rattus norvegicus* (rat), the sequence described in the GenEMBL database with accession number U18366, in its version 4 dated 12.01.2009, corresponding to the polynucleotide which codes for cardiotrophin 1 of *Mus musculus* (mouse), the sequence described in the GenEMBL database with accession number AB125661, in its version 2 dated 12.01.2009, corresponding to the polynucleotide which codes for cardiotrophin 2 of *Mus musculus* (mouse).

Alternatively, the agents capable of inducing CT-1 activity include "polynucleotides which code for a functionally equivalent variant of CT-1". Said polynucleotides are all those polynucleotides capable of coding for a variant of the polypeptides with CT-1 activity, as defined above by their specific sequences, said variant having at least 60% identity with CT-1. Said polynucleotides result from previously defined polynucleotides by means of the insertion, deletion or substitution of one or several nucleotides with respect to the aforementioned sequences. Preferably, the polynucleotides which code for functionally equivalent variants of CT-1 are polynucleotides whose sequence allows them to hybridize in highly restrictive conditions with the aforementioned polynucleotides. Typical conditions of highly restrictive hybridization include incubation in 6 X SSC (1 X SSC: 0.15 M NaCl, 0.015 M sodium citrate) and 40% formamide at 42 °C during 14 hours, followed by one or several washing cycles using 0.5 X SSC, 0.1% SDS at 60°C. Alternatively, highly restrictive conditions include those comprising a hybridization at a temperature of approximately 50°-55° C in 6 X SSC and a final washing at a temperature of 68° C in 1-3 X SSC. Moderate restrictive conditions comprise hybridization at a temperature of approximately 50° C until around 65° C in 0.2 or 0.3 M NaCl, followed by washing at approximately 50° C until around 55° C in 0.2 X SSC, 0.1% SDS (sodium dodecyl sulphate).

Preferably, when the agent capable of inducing CT-1 activity is a polynucleotide, it is operatively associated to a regulating region of the expression. The regulating sequences of use for the present invention may be sequences of nuclear promoters or, alternatively, enhancer sequences and/or other regulating sequences which increase the expression of the heterologous sequence of nucleic acid. The promoter may be constitutive, tissue specific or inducible. If constant expression of the heterologous sequence of nucleic acid is desired, then a constitutive promoter is used. Examples of well-known constitutive promoters include the immediate early promoter of cytomegalovirus (CMV), promoter of Rous sarcoma virus, and similar. Numerous other examples of constitutive promoters are well-known in the state of the art and can be used in the practice of the invention. If one wants the controlled expression of the heterologous sequence of nucleic acid, then an inducible promoter must be used. In a non-induced state, the inducible promoter is "silent". "Silent" wants to say that in the absence of an inducer little or no expression of the heterologous sequence of nucleic acid is detected; in the presence of an inducer, however, the expression of the heterologous sequence of nucleic acid occurs. Frequently, it is possible to control the expression level varying the concentration of the inducer. Controlling the expression, for example by varying the concentration of the inducer so that an inducible promoter is more strongly or weakly stimulated, it is possible to affect the concentration of the transcript product of the heterologous sequence of nucleic acid. In the case wherein the heterologous sequence of nucleic acid codes for a gene, it is possible to control the quantity of protein synthesized. In this way, it is possible to vary the concentration of a therapeutic product. Examples of well-known inducible promoters are: an estrogen or androgen promoter, a metallothionein promoter, or a promoter which responds to ecdysone. Other numerous examples are well known in the state of the art and can be used in the practice of the invention. In addition to the constitutive and inducible promoters (which usually function in a great variety of cell or tissue types) specific tissue promoters can be used to achieve expression of the heterologous sequence of nucleic acid specific in cells or tissues.

In another embodiment of the invention, the compound which induces cardiotrophin-1 activity is a vector comprising a polynucleotide as previously defined, i.e., which codes for CT-1 or a functionally equivalent variant of CT-1 which has, at least, 60% identity with CT-1. In an embodiment, the functionally equivalent variant of CT-1 has, at least, 60%, 70%, 80%, 85%, 90%, 92%, 94%, 96%, 98%, 99% identity with CT-1. Suitable vectors for the insertion of said polynucleotides are vectors derived from expression vectors in prokaryotes such as pUC18, pUC19, pBluescript and their derivatives, mp18, mp19, pBR322, pMB9, ColE1, pCR1, RP4, phages and "shuttle" vectors such as pSA3 and pAT28, expression vectors in yeasts such as vectors of the 2-micron plasmid type, integration plasmids, YEP vectors, centromeric plasmids and similar, expression vectors in insect cells such as the vectors of the pAC series and of the pVL series, expression vectors in plants such as vectors of the series pIBI, pEarleyGate, pAVA, pCAMBIA, pGSA, pGWB, pMDC, pMY, pORE and similar and expression vectors in superior eukaryote cells based on viral vectors (adenovirus, virus associated to adenovirus as well as retrovirus and, in particular, lentivirus) as well as non-viral vectors such as pSilencer 4.1-CMV (Ambion), pcDNA3, pcDNA3.1/hyg, pHCMV/Zeo, pCR3.1, pEFl/His, pIND/GS, pRc/HCMV2, pSV40/Zeo2, pTRACER-HCMV, pUB6/V5-His, pVAX1, pZeoSV2, pCI, pSVL, pKSV-10, pBPV-1, pML2d and pTDT1.

In another embodiment, the compound which induces cardiotrophin-1 activity is a cell capable of secreting into the medium cardiotrophin-1 or a functionally equivalent variant thereof which has, at least, 60% identity with CT-1. In an embodiment, the functionally equivalent variant of CT-1 has, at least, 60%, 70%, 80%, 85%, 90%, 92%, 94%, 96%, 98%, 99% of identity with CT-1. Suitable cells for the expression of cardiotrophin-1 or of the functionally equivalent variant thereof include, without limitation, cardiomyocytes, adipocytes, endothelial cells, epithelial cells, lymphocytes (B and T cells), mastocytes, eosinophils, vascular intima cells, primary cultures of isolated cells of different organs, preferably of cells isolated from Langerhans islets, hepatocytes, leukocytes, including mononuclear leukocytes, mesenchymal, umbilical cord or adult (of skin, lung, kidney and liver), osteoclasts, chondrocytes and other connective tissue cells. Cells of established lines such as Jurkat T cells, NIH-3T3, CHO, Cos, VERO, BHK, HeLa, COS, MDCK, 293, 3T3 cells, C2C12 myoblasts and W138 cells are also suitable.

Persons skilled in the art will appreciate that the cells capable of secreting into the medium cardiotrophin-1 or a functionally equivalent variant thereof may be found forming microparticles or microcapsules so that the cells have a greater useful life in patients. Materials suitable for the formation of microparticles object of the invention include any biocompatible polymeric material which permits continuous secretion of the therapeutic products and which acts as support of the cells. Thus, said biocompatible polymeric material may be, for example, thermoplastic polymers or hydrogen polymers. Among the thermoplastic polymers we have acrylic acid, acrylamide, 2-aminoethyl methacrylate, poly(tetrafluoroethylene-cohexafluorpropylene), methacrylic-(7-cumaroxy) ethyl ester acid, N-isopropyl acrylamide, polyacrylic acid, polyacrylamide, polyamidoamine, poly(amino)-p-xylylene, poly(chloroethylvinylether), polycaprolactone, poly(caprolactone-co-trimethylene carbonate), poly(carbonate urea) urethane, poly(carbonate) urethane, polyethylene, polyethylene and acrylamide copolymer, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic glycolic acid), poly(L-lactic acid), poly(gamma-methyl, L-glutamate), poly(methylmethacrylate), poly(propylene fumarate), poly(propylene oxide), polypyrrole, polystyrene, poly(tetrafluoroethylene), polyurethane, polyvinyl alcohol, polyethylene of ultra-high molecular weight, 6-(p-vinylbenzamide)-hexanoic acid N-p-vinybenzyl-D-maltonamide and copolymers containing more than one of said polymers. Among the polymers of hydrogel type we have natural materials of alginate, agarose, collagen, starch, hyaluronic acid, bovine serum albumin, cellulose and their derivatives, pectin, chondroitin sulphate, fibrin and fibroin, as well as synthetic hydrogels such as Sepharose® and Sephadex®.

It is known in the state of the art that some of the aforementioned polymers are rather unstable and tend to lose their character of gel, in addition to being relatively porous, which gives the result that the antibodies may access their inside and damage the cells. For these reasons, optionally, the microparticle of the invention may be surrounded by a semipermeable membrane which gives stability to the particles and which forms a barrier impermeable to antibodies. Semipermeable membrane is understood to be a membrane which permits the entry of all those solutes necessary for cell viability and which allows the exit of the therapeutic proteins produced by the cells contained within the microparticle, but which is substantially impermeable to antibodies, so that the cells are protected from the immune response produced by the organism housing the microparticle. Suitable materials to form the semipermeable membrane are materials insoluble in biological fluids, preferably polyamino acids, such as, for example, poly-L-lysine, poly-L-ornithine, poly-L-arginine, poly-L-asparagine, poly-L-aspartic, poly-benzyl-L-aspartate, poly-S-benzyl-L-cysteine, poly-gamma-benzyl-L-glutamate, poly-S-CBZ-L-cysteine, poly-ε-CBZ-D-lysine, poly-δ-CBZ-DL-ornithine, poly-O-CBZ-L-serine, poly-O-CBZ-D-tyrosine, poly(γ-ethyl-L-glutamate), poly-D-glutamic, polyglycine, poly-γ-N-hexyl-L-glutamate, poly-L-histidine, poly (α,β-[N-(2-hydroxyethyl)-DL-aspartamide]), poly-L-hydroxyproline, poly (α,β-[N-(3-hydroxypropyl)-DL-aspartamide]), poly-L-isoleucine, poly-L-leucine, poly-D-lysine, poly-L-phenylalanine, poly-L-proline, poly-L-serine, poly-L-threonine, poly-DL-tryptophan, poly-D-tyrosine or a combination thereof.

The compounds which induce cardiotrophin-1 activity are suitable for the preparation of a medicament for the prevention and/or treatment of acute kidney injury.

"Medicament" is understood as a pharmaceutical composition comprising a compound which induces cardiotrophin-1 activity (CT-1) according to the invention.

"Prevention" is understood as the administration of a compound which induces cardiotrophin-1 activity (CT-1) according to the invention, or a medicament containing it at an initial or early stage of the disease, or preferably to avoid its appearance. In a preferred embodiment of the invention, the compound which induces cardiotrophin-1 activity is used for the prevention of acute kidney injury.

The term "treatment" is used to designate the administration of a compound which induces cardiotrophin-1 activity (CT-1) according to the invention or of a medicament containing it to control the progression of the disease before or after the clinical signs have appeared. The control of the progression of the disease is understood as the desired or clinically beneficial results that include, but are not limited to, a reduction in the symptoms, reduction in duration of the disease, stabilization of pathological conditions (specifically avoiding additional deterioration), delaying the progression of the disease, improving the pathological condition and remission (both partial and total). The control of the progression of the disease also involves an extension in survival, compared with the expected survival if the treatment was not applied.

The term "acute kidney injury" (AKI) as used in the present document, is understood to be all kind of damage in the anatomic structures of the kidneys or the alteration of the perfusion thereof which leads to Acute kidney failure (AKF), i.e. which causes the sudden loss of kidney function, understanding as such the capacity of the kidneys to eliminate the waste and concentrate the urine without losing electrolytes. In an embodiment, AKF is understood as a disorder as defined by Mehta RL, et al. Crit Care. 2007;11:R31, wherein it is considered that AKF appears when at least one alteration of the kidney function is observed which leads in a time equal to or less than 48 h to
a) increased serum creatinine levels (absolute, ≥ 0.3 mg/dL; percentage, ≥ 50%; or an increase of 1.5 times on the basal levels),or
b) oliguria (< 0.5 mL/kg/h during more than 6 hours)

Another system also used to define AKF, which describes the severity of the kidney dysfunction based on an increase in serum creatinine levels and a decrease in urine elimination, is the RIFLE criterion (Bellomo R, et al. Crit Care. 2004;8: R204-R212; Bellomo R, et al. Intensive Care Med. 2004; 30:33 -37; Kellum JA, et al. Curr Opin Crit Care. 2002;8:509 -514).

According to the initial causes of acute kidney injury, this may be divided in pre-renal, renal and post-renal acute kidney injury.

In an embodiment, acute kidney injury is selected from the group of acute kidney injury of pre-renal cause and acute kidney injury of renal cause; preferably it is acute kidney injury of renal cause.

"Acute kidney injury of pre-renal cause" is understood as a kidney injury secondary to the reduction in normal perfusion in the kidney, which may be produced by depletion of the volume due to renal or extrarenal losses, the sequestration of fluids or to unsuitable perfusion pressures secondary to cardiac failures, cirrhosis or sepsis. It may also be the result of renal ischemia secondary to the aortic or renal artery clamping during abdominal surgery or during a kidney transplant (damage due to ischemia/reperfusion). Said damage is reversible if one acts early on the causes. The most-studied experimental model of acute kidney injury of pre-renal cause is the short clamping of the renal arteries in rodents producing damage due to ischemia/reperfusion. Table 1 shows a non-limiting list of the possible causes of acute kidney injury of pre-renal cause.

**Table 1. Aetiology of acute kidney injury of pre-renal cause.**

| |
|---|
| HYPOVOLEMIA: haemorrhages (gastrointestinal, surgical, postpartum); digestive (vomiting, diarrhoea); renal losses (diuretic, diabetic ketoacidosis, diabetes insipidus, suprarenal insufficiency); sequestration of liquids in the extravascular space (pancreatitis, peritonitis, bums, hypoalbuminemia) |
| DECREASE IN CARDIAC OUTPUT: Acute cardiac insufficiency (heart attack, tamponage, arrhythmias); massive pulmonary embolism; pulmonary hypertension. |
| PERIPHERAL VASODILATATION: Sepsis, anaphylaxia, antihypertensive, anaesthesia. |
| RENAL VASOCONSTRICTION: hypercalcemia, norepinephrine, Cyclosporin,amphotericin B, cirrhosis with ascites (hepatorenal syndrome) |
| ALTERATION IN RENAL AUTO-REGULATORY RESPONSES: Prostaglandin inhibitors, such as non-steroidal anti-inflammatory drugs (NSAIDs); angiotensin-converting enzyme (ACE) inhibitors |

"Acute kidney injury of renal cause" is understood as an intrinsic kidney injury, in the anatomic structures of the renal system, which can be classified anatomically as tubular, interstitial, glomerular and vascular.
- Tubular damage: acute tubular necrosis (ATN) is the lesion of the kidney tubules by ischemic or toxic mechanisms, i.e. it is the result of prolonged exposure to a pre-renal medium (ischemia) or the direct damage of toxins (nephrotoxins). The most frequent cause is ischemia, and it occurs when the causes of the pre-renal AKI are maintained prolonged over time. When the cause is a nephrotoxin, the most frequently involved are antibiotics (aminoglycosides, cephalosporins), radiological contrasts, NSAID, anaesthetics, endogenous toxins (myoglobinuria due to rhabdomyolysis, haemoglobinuria due to haemolysis, hyperuricemia, hypercalcemia). The classic "self-limiting" evolution of the ATN is a sudden increase in serum creatinine levels (damage stage), followed by stabilization (plateau stage) and finally a decrease in these measurements during 7 to 21 days (recovery stage). This pattern is correlated with the damage and the death of the tubular cells, their regeneration and the recovery of kidney tubular function. However, fluctuations in serum creatinine levels depend on many variables and are therefore not present in all cases of ATN. The most widely used experimental models of ATN are the administration to rats of nephrotoxic drugs, such as aminoglycosides or cisplatin, or the administration of chemical compounds such as uranyl nitrate (López- Novoa JM, et al. Kidney Int. 2011;79:33-45).
- Interstitial damage: acute interstitial nephritis (AIN) is caused by a depressed kidney function related to fever, eruptions, leukocytosis and eosinophilia. Acute interstitial nephritis is typically induced by drugs such as non-steroidal anti-inflammatory drugs (NSAIDs), antibiotics, diuretics, etc. although some infections (legionella, leptospira, cytomegalovirus, candida) and neoplastic disorders have also been associated to this condition. The most widely-used experimental model is the administration of NSAID (indomethacin) to rats (Varghese J, et al. Eur J Pharmacol. 2009; 614:114-121).
- Glomerular damage: the presence of erythrocytes, proteinuria or both suggests the existence of a glomerular disease. Hematuria, the presence of erythrocytes, hypertension and moderate proteinuria suggest a nephritic process (i.e. acute glomerulonephritis). Other causes of glomerular damage are malignant hypertension, vasculitis, haemolyticuremic syndrome, thrombotic thrombocytopenic purpura, pregnancy toxemia and sclerodermia. The most widely studied experimental model of acute glomerulonephritis is the administration of anti-Thy antibodies (mesangioproliferative nephritis) or adriamycin to rats.
- Vascular damage: acute kidney injury secondary to a vascular disorder may be difficult to diagnose. Causes of this type of damage are obstructions of renal arteries due to atherosclerotic plaque, thrombosis or embolia; and obstruction of renal veins due to thrombosis or compression. There are no well-known experimental models of this type of damage.

"Acute kidney injury of post-renal cause" is understood as kidney injury as a consequence of obstructions in the urinary tract which take place therein or intrarenal (stones, deposit of crystal, clots, tumours) or outside the urinary system or extrarenal (tumours, retroperitoneal fibrosis, lithiasis) and which prevent the exit of the urine formed, causing an increase in pressure which is transmitted retrogradely, compromising the glomerular filtrate. The model of acute kidney injury of post-renal cause most widely studied is unilateral urethral ligation in rats (Grande MT and López-Novoa JM. Nat Rev Nephrol. 2009; 5:319-328). In another embodiment, the acute kidney injury is of post-renal cause.

In a preferred embodiment, the acute kidney injury is of pre-renal cause, preferably selected from acute kidney injury caused by exposure of a subject to ischemia and acute kidney injury caused by ischemia followed by reperfusion.

"Acute kidney injury caused by exposure of a subject to ischemia" is understood as the kidney injury caused in a subject as a consequence of ischemia of the renal tissue. The term "ischemia" makes reference to tissue damage and inflammatory response caused in the kidney as a consequence of the transitory decrease in blood flow and consequent decrease in the supply of oxygen, nutrients and the elimination of metabolism products in said organs.

"Acute kidney injury caused by ischemia followed by reperfusion" is understood as the kidney injury caused in a subject as a consequence of the transitory decrease in blood flow (ischemia) and of the subsequent re-establishment of said blood flow in the organ after the period of ischemia (reperfusion).

In another preferred embodiment of the invention, the acute kidney injury is any acute kidney injury with the exception of that caused by ischemia or by ischemia/reperfusion.

In a preferred embodiment of the invention, the acute kidney injury is of renal cause, preferably caused by a nephrotoxic agent.

In an even more preferred embodiment of the invention, the acute kidney injury is caused by a nephrotoxic agent.

"Nephrotoxic agent", in the context of the present invention, is understood as any substance capable of producing toxicity in the renal system, i.e. any substance which, situated in the renal system is capable of producing disturbances and imbalances in its morphological and physiological aspects which lead to organ lesion. The nephrotoxic agents include, without limitation, pharmacological agents, diagnostic agents and toxic agents such as environmental chemical substances, animal and plant toxins, drugs of abuse, etc. The nephrotoxic pharmacological agents contemplated by the present invention include, without limitation, analgesics (paracetamol, phenacetin, dipyrones) and non-steroidal anti-inflammatory drugs (aspirin, indomethacin); antibiotics such as cephalosporins (cefaloridine), tetracyclines, vancomycin, carbapenems (imipenem), polymyxin B, rifampicin and aminoglycosides, viomycin and capreomycin, sulfonamides, pentamidine; antifungal agents (amphotericin B); chemotherapy and antineoplastic agents such as cisplatin, cyclofosfamide, methotrexate, 5-fluorouracil; immunosuppressive agents such as cyclosporin A and interactions of pharmacological agents. The nephrotoxic diagnostic agents include, without limitation, radiocontrast or radiological contrast agents (iodine hippurate, ionic contrasts) or image diagnosing agents. The toxic agents that affect the renal system include, without limitation, environmental chemical substances including halogenated hydrocarbons used as herbicides, metals and especially heavy metals (mercury, cadmium, lead, chromium), mycotoxins, organic solvents (petrol, trichloroethylene, carbon tetrachloride, ethylene glycol), pesticides (paraquat, derivatives of phenoxyacetic acid, arsenic, strychnine, potassium chlorate and sodium borate), silicon oxide; animal toxins from ophidia (Tiger Snake, Russell's viper or Rattle-Snake viper), arachnids (*Loxosceles Ruferens* or brown or corner spider); toxic vegetables and plants (*Daphne mezereum, Actanea spicata, Juniperus communis, Rhamnus franqula, Rhamnus catharticus, Ricinus commnunis*), fungi (*Amanita Phalloides*); drugs of abuse (heroin, cocaine); virus and protozoa (hantavirus, *Plasmodium malariae, Plasmodium falciparum*).

In an even more preferred embodiment, the nephrotoxic agent is selected from the group of contrast agents, antibiotics, immunosuppressive agents and antineoplastic agents.

Given the large number of processes and diagnostic studies which require the use of contrast media, a great percentage of the population is today at risk of suffering contrast-induced nephropathy.

The expression "contrast-induced nephropathy", in the context of this invention, relates to kidney injury produced by the administration of contrast agents. This is the third cause of kidney injury in the hospital setting and is defined by an increase in serum creatinine levels of 25% or greater (0.5 mg/dl) within 72 hours of administration of a contrast medium (Solomon R. et al. N Engl J Med. 1994; 331:1416-1420; Briguori C. et al. Circulation. 2007; 115:1211-1217). Associated risk factors are advanced age, diabetes, chronic kidney disease, multiple myeloma and volume depletion. The most widely studied experimental model of contrast-induced nephropathy is the administration of radiocontrast media to sensitized rats. The sensitization may occur previously by depletion of the extracellular volume or the administration of a nephrotoxin at low doses. Therefore, in another aspect, the method of the invention is carried out in subjects sensitized to suffering AKI. In a preferred embodiment, the subjects presensitized to suffering AKI have been treated with subtoxic doses of a nephrotoxic agent. In a preferred embodiment, the nephrotoxic agent with which the subject has been treated is an antibiotic. In a preferred embodiment, the antibiotic is selected from the group of aminoglycosides and cephalosporins. In an even more preferred embodiment, the aminoglycoside antibiotic is selected from the group of gentamicin, tobramycin, amikacin, netilmicin, kanamycin, streptomycin, sisomycin, neomycin and combinations thereof.

In an embodiment of the invention, the nephrotoxic agent is a contrast agent.

The term "contrast agent", in the context of the present invention, relates to a biocompatible compound which may be detected after being administered to an individual and which may be used in *in vivo* diagnostic imaging methods. The use of said contrast agents improves the visibility of structures or fluids within the body, facilitating the differentiation of different parts of the image and increasing the "contrast" between those different regions. The term "contrast agent" thus covers agents which are used to increase the quality of an image which can be generated, however, in the absence of said agent (as is the case, for example, in magnetic resonance), as well as agents which are prerequisites for image generation (as is the case, for example, of scintigraphy). Suitable contrast agents include, without limitation, contrast agents for scintigraphy, for single-photon emission computed tomography (SPECT), for positron emission tomography (PET), for Raman spectroscopy, for magnetic resonance diagnostic imaging (MR) and for optical imaging diagnosis. Said contrast agents are administered by the routes that are best distributed by the structure when being examined, whether consumed or by enema in the case of the digestive tract, inhaled by the airways or injected to view the blood vessels, organs and tissues.

The contrast agents include radiodrugs that are normally marked with positron emitters such as ¹¹C, ¹³N, ¹⁵O, ¹⁸F, ⁸²Rb, ⁶²Cu and ⁶⁸Ga. SPECT uses mainly y emitters, such as ¹²³I, ^{99m}Tc and ¹¹¹In. The categories of scintigraphy (positron emission tomography (PET), single-photon emission computed tomography (SPECT)) are image diagnosing techniques of cross-sections that map the location and concentration of radiotracers marked with radionuclides. PET and SPECT can be used to locate and characterize a radionuclide measuring the metabolic activity. PET and SPECT provide information related to information at a cellular level such as cell viability. In PET, a patient consumes or is injected with a slightly radioactive substance that emits positrons, which can be followed as the substance passes through the body. In a common application, for example, patients are administered glucose with attached positron emitters, and their brains are controlled as they perform different tasks. Since the brain uses glucose when it works, a PET image shows in what areas brain activity is high. In certain embodiments of the invention, a cell is marked *ex vivo* for PET or SPECT *in vivo* image diagnosing. Highly related to PET is single-photon emission computed tomography or SPECT. The main difference between the two is that instead of a positron emitting substance, SPECT uses a radioactive tracer which emits low energy photons.

The contrast agents for CT images include, for example, iodinated or brominated contrast media. The examples of these agents include iothalamate, iohexol, diatrizoate, iopamidol, ethiodol or iopanoate. Gadolinium agents have also been described for their use as CT contrast agents (see, for example, Henson JW, et al. AJNR Am J Neuroradiol. 2004; 25: 969-72). For example, gadopentate agents have been used as CT contrast agents (discussed in Strunk HM and Schild H. Eur Radiol. 2004; 14: 1055-62). Computerized tomography (CT) is considered as a form of image diagnosing. Taking a series of X-rays, sometimes more than one thousand from several angles and then combining them with a computer, CT makes possible to build a three-dimensional image of any part of the body. A computer is programmed to show two-dimensional sections from any angle and at any depth. In CT, the intravenous injection of a radio-opaque contrast agent, such as that described here, may aid in the identification and outlining of soft tissue masses when the initial CT scanners are not diagnostic.

The contrast agents for optical images include, for example, fluorescein, a fluorescein derivative, indocyanine green, Oregon green, a derivative of Oregon green, rhodamine green, a derivative of rhodamine green, an eosin, an erythrosine, Texas red, a derivative of Texas red, malachite green, Nanogold® mono(sulfosuccinimidyl ester), cascade blue, a derivative of coumarin, a naphthalene, a derivative of pyridyloxazole, cascade yellow dye, dapoxyl die and several other fluorescent compounds disclosed here.

The contrast agents for magnetic resonance include, without limitation, complexes of metals selected from the group consisting of chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and erbium (III).

The invention is especially aimed at those nephrotoxic contrast agents and, therefore, potential causal factors of acute kidney injury.

In a preferred embodiment, the contrast agent is selected from the group of a contrast agent containing iodine, a contrast agent containing bromine, a contrast agent containing barium, a contrast agent containing gadolinium and combinations thereof.

In a preferred embodiment the contrast agent contains iodine. The contrast agents containing iodine or iodinated contrast agents are used, in general, for radiological examinations such as elimination pyelography, hysterosalpingography, phlebography, CT angiography, angio CT and arthro CT, among others, as intravenous radiocontrasts. Iodinated contrast agents contemplated by the present invention include, without limitation, ionic monomers (diatrizoic acid or amidotrizoic acid and their salts, metrizoic acid and its salts, iothalamic acid and its salts, iodamide, ioxitalamic acid and its salts, iopanoic acid and its salts, ioglicic acid and its salts, acetrizoic acid and its salts, iocarmic acid and its salts, methiodal), ionic dimers (ioxaglic acid and its salts, iotroxic acid and its salts), non-ionic monomers (iohexol, iopamidol, ioversol, iobitridol, iopromide, metrizamide, iopentol), non-ionic dimers (iodixanol, iotrolan, ioxilan, iomeprol) and water-insoluble (propyliodone). The present invention also includes the combinations of said compounds and/or their pharmaceutically acceptable salts. Suitable salts for said iodinated contrast agents are, without limitation, any type of pharmaceutically acceptable salt, but preferably sodium or meglumine salt. Examples of non-limitative salts of iodinated contrast agents contemplated by the present invention are diatrizoate sodium, diatrizoate meglumine, metrizoate sodium, metrizoate meglumine, iothalamate sodium, iotalamate meglumine, ioxaglate sodium, ioxaglate meglumine, etc.

In a preferred embodiment of the invention, the contrast agent containing iodine is selected from the group of diatrizoic acid (or amidotrizoic acid) and its salts, metrizoic acid and its salts, ioxaglic acid and its salts, iothalamic acid and its salts, iopamidol, iohexol, ioxilan, iopromide, ioversol, iodixanol, metrizamide and combinations thereof; preferably it is one or more diatrizoate salts (also known as amidotrizoate). Amidotrizoates are used in a wide variety of processes such as urography and exploration of the gall bladder, bile ducts and the spleen. A mixture of diatrizoate salts is usually preferred such as amidotrizoate sodium and amidotrizoate meglumine to minimize adverse effects and improve the quality of the exploration. In an even more preferred embodiment the contrast agent containing iodine is a combination of diatrizoate sodium and diatrizoate meglumine.

In another embodiment the contrast agent contains bromine. The contrast agents containing bromine or brominated contrast agents included in the present invention are, without limitation, contrast agents such as those disclosed in patent applications WO93/08122, EP 1186305 A1, FR 2736051 A1, EP 073715 A1, EP 074307 A1, EP 074309 A1, EP 0118348 A1, FR 2541272 A1; brominated fluorocarbons such as perfluorooctyl bromide (C8BrF17), perfluorohexyl bromide. The invention also contemplates mixtures of said contrast agents containing bromine and/or pharmaceutically acceptable salts thereof.

In another embodiment the contrast agent contains barium, preferably barium sulfate. Barium sulphate is a metal salt which is used to define the gastrointestinal tract. It can be used in single or double contrast tests or in computer-assisted axial tomography.

In another embodiment the contrast agent contains gadolinium. The contrast agents containing gadolinium are, without limitation, gadopentetic acid, gadodiamine, gadoteric acid, gadoteridol, gadopentate, gadodiamide, gadobenic acid, gadofosveset, gadoversetamide, gadoxetic acid, gadobutrol, gadocoletic acid and gadodenterate. The present invention also contemplates the salts of said contrast agents containing gadolinium, being suitable any type of pharmaceutically acceptable salts, preferably sodium salt and meglumine salt.

In another embodiment the nephrotoxic agent is an antibiotic. "Antibiotic" is understood as a chemical substance produced by a living being or a synthetic derivative thereof which at low concentrations kills or prevents the growth of certain classes of sensitive microorganisms, generally bacteria, although some antibiotics are also used for the treatment of infections by fungi or protozoa. Antibiotics are used in human, animal or horticultural medicine to treat infections caused by microorganisms. Antibiotics included in the present invention are, without limitation, aminoglycoside antibiotics, ansamycins, carbacefem, carbapenems, cephalosporins, glycopeptides, macrolides, monobactams, penicillins, polypeptides, quinolones, sulfonamides, tetracyclines and others such as arsphenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin or rifampicin, tinidazole, viomycin and capreomycin; preferably cephalosporins, tetracyclines, glycopeptides, carbapenems, polypeptides, rifampicin, aminoglycosides, sulfonamides, viomycin and capreomycin. In a preferred embodiment the antibiotic is selected from the group of aminoglycosides and cephalosporins.

"Aminoglycoside antibiotics" are understood as bactericide antibiotics which act at a level of ribosomes in the 30S bacterial subunit and, hence, at a level of protein synthesis, creating porosities in the external membrane of the bacterial cell wall. Aminoglycoside antibiotics included by the present invention are, without limitation, gentamicin, amikacin, spectinomycin, trospectomycin, streptomycin, neomycin, gentamicin, tobramycin, amikacin, netilmicin, sisomycin, paromomycin and kanamycin. The present invention also includes the pharmaceutically acceptable salts of said antibiotics. In a preferred embodiment the aminoglycoside antibiotic is selected from the group of gentamicin, tobramycin, amikacin, netilmicin, kanamycin, streptomycin, sisomycin, neomycin and combinations thereof; it is preferably gentamicin.

"Cephalosporins" are understood as a class of beta-lactam antibiotics derived from 7- cephalosporanic acid and which act interfering in the synthesis of peptidoglycan of the bacterial cell wall and inhibiting the final transpeptidation, necessary for crosslinking, which generates a bactericide effect. In the context of the present invention the term cephalosporins includes, for example and without limitation, first generation cephalosporins (cefadroxil, cefazolin, cefalotin, cefalexin), second generation cephalosporins (cefaclor, cefamandole, cefoxitin, cefprozil, cefuroxime), third generation cephalosporins (cefixime, cefdinir, cefditoren, cefoperazone, cefotaxime, cefpodoxime, ceftazidime, ceftibuten, ceftizoxime, ceftriazone), fourth generation cephalosporins (cefepime) and fifth generation cephalosporins (ceftobiprole). The present invention also includes the pharmaceutically acceptable salts of said cephalosporins.

In another embodiment the nephrotoxic agent is an immunosuppressive agent. "Immunosuppressive agent" is understood as a substance which produces the immunosupression of the immune system. Said immunosuppressive agents are useful for preventing the rejection of transplanted organs and for the treatment of autoimmune diseases or diseases that may be of autoimmune origin, such as vasculitis, rheumatoid arthritis, ulcerative colitis, psoriasis or systemic erythematous lupus. Immunosuppressive agents included in the present invention are, without limitation, cytostatic agents such as azathioprine, cyclofosfamide, methotrexate, mycophenolate mofetil; glucocorticoids such as prednisone; calcineurin enzyme inhibitors such as cyclosporin A, tacrolimus (FK506); rapamycin or sirolimus; everolimus; polyclonal antibodies such as thymoglobulin; monoclonal antibodies such as rituximab, daclizumab, etanercept. The present invention also includes the pharmaceutically acceptable salts of said immunosuppressive agents. In a preferred embodiment the immunosuppressive agent is selected from cyclosporin A, tacrolimus (FK506) and everolimus; preferably cyclosporin A.

In another embodiment the nephrotoxic agent is an antineoplastic agent. "Antineoplastic agent" is understood as a substance which prevents the development, growth or proliferation of malignant tumour cells, and which may be of natural, synthetic or semisynthetic origin. Antineoplastic agents contemplated in the present invention include, without limitation, DNA alkylating agents; platinum-based compounds; antimetabolites; antitumoral antibiotics; topoisomerase I and II inhibitors; enzymes such as L-asparaginase; vinca alkaloids; taxanes; hormonal agents such as anti-estrogenics, aromatase inhibitors, LH-RH analogues, antiandrogenics and glucocorticoids; other agents such as interleukins, interferons, monoclonal antibodies and BCG vaccine.

In a preferred embodiment the antineoplastic agent is selected from the group of a platinum-based compound, an antimetabolite, a DNA alkylating agent, a topoisomerase I or II inhibitor, and a combination thereof.

"Platinum-based compound", as is used in the present document, is understood as any compound containing a platinum atom capable of bonding to and intercalating in the DNA, inducing the repair activation of the DNA and finally triggering apoptosis. Platinum-based compounds include, without limitation, carboplatin, cisplatin [cis-diaminodichloroplatinum, (CDDP)], oxaliplatin, iproplatin, nedaplatin, triplatin tetranitrate, tetraplatin, satraplatin (JM216), JM118, JM149, JM335, transplatin, ZD0473, cis, trans, cis-Pt(NH3)(C6H11NH2)(OOCC3H7)2Cl, malanate-1,2-diaminocyclohexane-platinum (II), 5-sulfosalicylate-trans-(1,2-diaminocyclohexane)platinum (II) (SSP), poly-[(trans-1,2-diaminocyclohexane)platinum]- carboxyamylose (POLY-PLAT), 4-hydroxy-sulfonylphenylacetate (trans-1,2-diaminocyclohexane) platinum (II) (SAP) and similar. In a preferred embodiment the platinum-based compound is selected from the group of cisplatin, carboplatin and a combination thereof; preferably, it is cisplatin.

The term "antimetabolite", as used in the present document, refers in its widest sense to substances that disturb the normal metabolism and substances that inhibit the electron transfer system to prevent the production of intermediaries rich in energy, due to their structural or functional similarities with metabolites which are important for live organisms (such as vitamins, coenzymes, amino acids and saccharides). Antimetabolites suitable for their use in the present invention include, without limitation, folic acid antimetabolites (aminopterin, denopterin, methotrexate, edatrexate, trimetrexate, nolatrexed, lometrexol, pemetrexed, raltitrexed, piritrexim, pteropterin, leucovorin, 10-propargyl-5,8-dideazafolate (PDDF, CB3717)), ribonucleotide inhibitors (hydroxyurea), purine analogues (cladribine, clofarabine, fludarabine, mercaptopurine, pentostatin, tioguanine) and pyrimidine analogues (capecitabine, 5-azacitidine, citarabine or ara-C, decitabine, fluorouracil, 5-fluorouracil, doxifluridine, floxuridine and gemcitabine). The present invention also contemplates the pharmaceutically acceptable salts of said antimetabolites. In a preferred embodiment the antimetabolite is selected from the group of methotrexate, pentostatin, 5-azacytidine, hydroxyurea and a combination thereof.

"DNA alkylating agent", is understood as an alkylating agent used in the treatment of cancer which is capable of adding an alkyl group to the DNA of cells which quickly divide, leading to stopping replication and to cell death.

DNA alkylating agents are nitrogen mustards, nitrosoureas, ethyleneimine derivatives, alkyl sulfonates and triazenes, including, but without being limited to, cyclofosfamide (Cytoxan™), busulfan, improsulfan, piposulfan, pipobroman, melphalan (L-sarcolysine), chlorambucil, mechlorethamine or mustine, uramustine or uracil mustard, novembichin, phenesterin, trofosfamide, ifosfamide, carmustine (BCNU), lomustine (CCNU), chlorozotocin, fotemustine, nimustine, ranimustine, semustine (methyl-CCNU), streptozotocin, thiotepa, triethylenemelamine, triethylenethiophosphoramine, mitomycin C, procarbazine, altretamine, dacarbazine, mitozolomide and temozolomide. The present invention also contemplates the pharmaceutically acceptable salts of said compounds. In a preferred embodiment the DNA alkylating agent is selected from the group of carmustine, lomustine, semustine, ifosfamide, mitomycin C and a combination thereof.

"Topoisomerase I or II inhibitor" is understood as an agent designed to interfere with the action of topoisomerase I and II enzymes. Topoisomerase I inhibitors include, without limitation, irinotecan, topotecan, camptothecin, acetylcamptothecin, 9-aminocamptothecin, lamellarin D and betulinic acid. Topoisomerase II inhibitors include, without limitation, amsacrine, etoposide, teniposide and doxorubicin. The present invention also contemplates the pharmaceutically acceptable salts of said compounds. In a preferred embodiment the topoisomerase I or II inhibitor is selected from the group of etoposide, teniposide, doxorubicin and a combination thereof.

The invention also contemplates combinations of all these antineoplastic agents, such as, for example and without limitation, cisplatin-paclitaxel, cisplatin-gemcitabine, cisplatin-docetaxel, carboplatin-paclitaxel, cisplatin-etoposide, carboplatin-etoposide, carboplatin-gemcitabine, carboplatin-docetaxel, cisplatin-teniposide, oxaliplatin gemcitabine, oxaliplatin-paclitaxel, cisplatin-paclitaxel-gemcitabine, cisplatin-doxorubicin-5-fluorouracil (AFP), cyclofosfamide-doxorubicin-cisplatin (CISCA) and cisplatin-fluorouracil (CF).

"Pharmaceutically acceptable salt", as used in the present document, relates to a salt recognized for its use in animals and, more particularly, in human beings, and includes the salts used to form base addition salts, whether inorganic, such as, for example and without being limiting in nature, lithium, sodium, potassium, calcium, magnesium, manganese, copper, zinc or aluminium, among others, or organic, such as, for example and without being limiting in nature, ethylamine, diethylamine, ethylenediamine, ethanolamine, diethanolamine, arginine, lysine, histidine or piperazine among others, or acid addition salts, either organic, such as, for example and without being limiting in nature, acetate, citrate, lactate, malonate, maleate, tartrate, fumarate, benzoate, aspartate, glutamate, succinate, oleate, trifluoroacetate, oxalate, pamoate or gluconate among others, or inorganic, such as, for example and without being limiting in nature, chloride, sulfate, borate or carbonate among others.

The administration of the compound which induces cardiotrophin-1 activity may be performed before the administration of the nephrotoxic agent, during the administration of said agent and/or after the administration thereof. Typically, the administration of the compound which induces cardiotrophin-1 activity is started before administration of the nephrotoxic agent (for example one day before) and continues until a few days after the end of administration of the nephrotoxic agent, for example until 4 days, until 5 days, until 6 days, until 7 days after the end of administration.

In a preferred embodiment of the invention, the compound which induces cardiotrophin-1 activity is co-administered together with the nephrotoxic agent. Coadministration implies that both the compound inducing cardiotrophin-1 activity and the nephrotoxic agent are administered at the same time, either in separate pharmaceutical compositions, in the same pharmaceutical composition, or when they are environmental nephrotoxic agents, the contact of said nephrotoxic agents with the subject treated coexists in time with administration of the compound which induces cardiotrophin-1 activity.

The administration of the compound which induces cardiotrophin-1 activity can be performed before the appearance of ischemia or ischemia-reperfusion, during it or once the ischemia or ischemia-reperfusion has ceased. Typically, the administration of the compound which induces cardiotrophin-1 activity is performed a little after the establishment of ischemia and is maintained throughout the ischemic period, stopping before the reperfusion starts.

The subject to which the compound which induces cardiotrophin-1 activity is administered may be any mammal and includes, but is not limited to, pets and farm animals, primates and humans, for example human beings, non-human primates, cows, horses, pigs, sheep, goats, dogs, cats or rodents. Preferably, the subject is a human being of female or male sex and of any race or age.

Persons skilled in the art will appreciate that in the medicament the active principles must be in a therapeutically effective quantity, and that he formulation of said active compounds shall be carried out depending on the type of administration. "Therapeutically effective quantity", as used here, is understood as the quantity of compound that allows totally or partially alleviating the symptoms associated with acute kidney injury or which prevent the progression or the worsening of symptoms, or which prevents the appearance of acute kidney injury in a subject at risk of suffering it. In a preferred embodiment of the invention cardiotrophin-1 is administered at a dose of 100 µg/kg per day.

In an embodiment of the invention, the compound which induces cardiotrophin-1 activity is administered by intravenous route.

The inventors have demonstrated that the compound which induces cardiotrophin-1 activity is capable of reducing the deterioration of renal excretory function in patients subjected to kidney injury, as demonstrated in Figures 4, 21 and 28B which show a reduction in the decrease of creatinine clearance; Figures 2, 3, 14, 19, 20 and 28A which show a reduction in the accumulation in the blood of metabolism products such as creatinine, urea and their derivatives; Figures 5 and 22, which show a decrease in the appearance of proteinuria. Therefore, in a preferred embodiment of the invention, the compound which induces cardiotrophin-1 activity reduces the deterioration of renal excretory function.

"Deterioration of renal excretory function" is understood as the complete or partial loss of the kidneys' capacity to eliminate wastes and concentrate the urine without losing electrolytes measured through an increase in serum creatinine levels, decrease in urine elimination, decrease in creatinine clearance, increase in urea and derivatives in serum, or the appearance of proteinuria. Methods suitable for determining these parameters have been previously described.

The authors of the present invention have demonstrated that the single administration of cardiotrophin-1 is sufficient to prevent and/or treat acute kidney injury. In a preferred embodiment of the invention, the administration of the compound which induces cardiotrophin-1 activity is performed in the absence of a soluble receptor of a member of the IL-6 family or a fragment thereof. In a preferred embodiment the soluble receptor is the CT-1 receptor or a fragment thereof.

"Soluble receptor of a member of the IL-6 family" is understood as a receptor in soluble form capable of bonding to a cytokine belonging to the interleukin 6 family forming a complex which is capable of directly interacting with gp130. The members of said family include, without limitation, leukemia inhibitory factor (LIF), cardiotrophin-1 (CT-1), the ciliary neurotrophic factor (CNTF), interleukin-11 (IL-11), oncostatin M (OSM), the cardiotrophin-like cytokine (CLC) and interleukin 6 (IL-6). "Fragment of a soluble receptor of a member of the IL-6 family" is understood as any fragment of a soluble receptor of a member of the IL-6 family which maintains the bonding capacity to cardiotrophin-1 and also the capacity of interacting with gp130.

"Soluble receptor of CT-1" is understood as a receptor in soluble form capable of bonding to cardiotrophin-1 forming a complex which is capable of directly interacting with gp130. "Fragment of a soluble receptor of CT-1" is understood as any fragment of the soluble receptor of CT-1 which maintains the capacity of bonding to cardiotrophin-1 and also the capacity of interaction with gp130.

The expression "is performed in the absence of a soluble receptor of a member of the IL-6 family or a fragment thereof" means that together with cardiotrophin-1 no soluble receptor of a member of the IL-6 family is administered exogenously nor a fragment thereof.

In another preferred embodiment of the invention, the administration of the compound which induces cardiotrophin-1 activity is performed in the absence of a metanephric mesenchymal growth factor.

"Metanephric mesenchymal growth factor" is understood as, in the context of the invention, any growth factor capable of stimulating the proliferation of the metanephric mesenchyma and includes, without limitation, TGFα, FGF-2, FGF-9, TIMP-1 and TIMP-2.

The expression "is performed in the absence of a metanephric mesenchymal growth factor" means that together with cardiotrophin-1 no metanephric mesenchymal growth factor is administered exogenously.

### COMPOSITIONS OF THE INVENTION

The authors of the present invention have demonstrated that the kidney injury produced by nephrotoxic agents may be counteracted by the administration of a compound which induces cardiotrophin-1 activity.

Therefore, another aspect of the invention is a composition comprising, together or separately, a compound which induces cardiotrophin-1 activity and a nephrotoxic agent.

The expression "compound which induces cardiotrophin-1 activity" has been described previously in the context of the uses of the invention and comprises, as in the first aspect of the invention, a compound selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii) and
(iv) a cell capable of secreting into the medium cardiotrophin-1 or a functionally equivalent variant thereof which has, at least, 60% identity with CT-1 wherein the different compounds have previously been described in detail.

The term "nephrotoxic agent" has been defined previously in the context of the uses of the invention and is selected from the group of contrast agents, antibiotics, immunosuppressive agents and antineoplastic agents, which have also been previously described.

The term "composition", as used in the present document, relates to a composition formed by at least two components: a compound which induces cardiotrophin-1 activity and a nephrotoxic agent, which may be a therapeutic agent or a contrast agent. Said components may be physically together, constituting a single formulation (for example, as a tablet or capsule comprising a fixed quantity of each one of the components) or, in contrast, they may be formulated separately to later combine them for their joint, sequential or separate administration. The compositions of the invention also contemplate the formulation as a "kit of parts" wherein the components are separately formulated but are packaged in the same container, and make it possible to combine them before their administration. Persons skilled in the art will appreciate that the formulation of the first and second component of the compositions of the invention may be similar, i.e. formulated similarly (in tablets), which allows their administration by the same route. If the different components of the composition of the invention are formulated separately, the two components can be presented in a blister. Each blister contains the drugs which must be consumed throughout the day. If the drugs must be administered several times a day, the drugs corresponding to each administration can be arranged in different sections of the blister, preferably recording in each section of the blister the time of day when they must be administered. Alternatively, the components of the composition of the invention may be formulated differently so that the different components are administered differently. Thus, it is possible, for example, that the first component is formulated as a tablet or capsule for its oral administration and that the second component is formulated for its intravenous administration.

The compositions of the invention are administered by the methods known by a person skilled in the art, including, without limitation, by intravenous, oral, nasal, parenteral, topical, transdermal, rectal and similar routes.

The relation between the components that form part of the compositions of the invention will depend on the CT-1 activity-inducing compound and the nephrotoxic agent used in each case in particular, as well as the desired indication.

The compositions useful in the practice of the method of the invention include a therapeutically effective quantity of a compound which induces cardiotrophin-1 activity and a therapeutically effective quantity of the nephrotoxic agent, when said agent is a therapeutically active drug. Furthermore, the compositions useful in the practice of the method of the invention include a therapeutically effective quantity of a compound which induces cardiotrophin-1 activity and a suitable quantity of the nephrotoxic agent to obtain a contrast image useful in diagnostic processes when said nephrotoxic agent is a contrast agent. The compositions of the invention also include a pharmaceutically acceptable carrier.

In a particular embodiment the composition is a pharmaceutical composition comprising, together or separately, a compound which induces cardiotrophin-1 activity, a nephrotoxic agent and at least one pharmaceutically acceptable excipient.

"Therapeutically effective quantity of a compound which induces cardiotrophin-1 activity" is understood as that quantity capable of producing one or more of the effects listed above and attributable to CT-1, and it can be determined by standard techniques based on the methods detailed in the present description.

"Therapeutically effective quantity of the nephrotoxic agent" is understood as that quantity of nephrotoxic agent necessary to produce a therapeutic effect and which varies depending on the therapeutic effect sought, i.e. it varies depending on whether the nephrotoxic agent is an antibiotic, an antineoplastic agent or an immunosuppressive agent.

The term "pharmaceutically acceptable" means approved by a regulatory agency of the federal or state government or included in the pharmacopoeia of the USA or another generally recognized pharmacopoeia, for use in animals, and more particularly in humans. The term "carrier" relates to a diluent, coadjuvant, excipient, or vehicle whereby the therapeutic compound is administered. Said pharmaceutical carriers may be sterile liquids, such as water and oils, including those of petroleum, animal, plant or synthetic origin, such as peanut oil, soy oil, mineral oil, sesame oil and similar. Suitable pharmaceutical excipients include starch, glucose, lactose, sucrose, gelatine, malt, rice, flour, chalk, silica gel, sodium stearate, glycerol monostearate, talc, sodium chloride, dried skimmed milk, glycerol, propylene, glycol, water, ethanol and similar. The composition, if desired, may also contain minor quantities of wetting or emulsifying agents, or pH buffering agents. These compositions may take the form of solutions, suspensions, emulsions, tablets, pills, capsules, powders, extended release formulations and similar. The composition may be formulated as a suppository, with binders and traditional carriers such as triglycerides. The oral formulation may include standard carriers such a pharmaceutical types of mannitol, lactose, starch, magnesium stearate, sodium sucrose, cellulose, magnesium carbonate, etc. Examples of suitable pharmaceutical carriers are described in "Remington's Pharmaceutical Sciences". 17th edition revised (December 1985). Editorial Mack Pub. Co.

The composition can be formulated in accordance with routine processes as a pharmaceutical composition adapted for intravenous, subcutaneous or intramuscular administration to human beings. When necessary, the composition may also include a solubilisation agent and a local anaesthetic such as lidocaine to relieve pain in the injection site. When the composition is going to be administered by infiltration, it can be dispensed with an infiltration vial containing pharmaceutical quality water or saline solution. When the composition is administered by injection, a water vial for injection or sterile saline solution can be provided, so that the ingredients can be mixed before administration.

The quantity of CT-1 activity inducing compound which will be effective in the treatment of kidney injury may be determined by standard clinical techniques based on the present description. Furthermore, *in vitro* assays can optionally be used to identify the optimum dosing intervals. The precise dose to be used in the formulation will also depend on the administration route and the severity of the condition, and must be decided in accordance with the physician's opinion and the circumstances of each subject.

For systemic administration, a therapeutically effective dose can be initially estimated from *in vitro* assays. For example, a dose can be formulated in animal models to achieve a circulating concentrating interval which includes the CI50 determined in cell culture. Said information can be used to more accurately determine the useful doses in humans. The initial doses can also be estimated from *in vivo* data, e.g. animal models, using techniques well known in the state of the art. Someone normally skilled in the art can easily optimize the administration to humans based on animal data.

Thus, the methods of prevention and/or treatment of acute kidney injury in accordance with the invention contemplate the joint, sequential or separate administration of the compounds with the capacity of inducing CT-1 activity with one or several nephrotoxic compounds. Therefore, in a preferred embodiment the composition is administered simultaneously, separately or sequentially.

All the particular embodiments of the uses of the present invention are also applicable to the compositions of the invention.

The compositions of the invention, containing a compound which induces cardiotrophin-1 activity, may be used to prevent acute kidney injury produced by the nephrotoxic component of said composition when this is administered with a therapeutic or diagnostic purpose.

Therefore, in another aspect, the invention relates to a composition comprising a compound which induces cardiotrophin-1 activity and a contrast agent for use as a diagnostic agent. In another aspect, the invention relates to a composition comprising a compound which induces cardiotrophin-1 activity and a contrast agent for the preparation of a diagnostic agent. In another aspect, the invention relates to the use of a composition comprising a compound which induces cardiotrophin-1 activity and a contrast agent for the preparation of a diagnostic agent. In another aspect, the invention relates to a diagnostic method by contrast in a patient comprising the administration to said patient of a composition comprising a compound which induces cardiotrophin-1 activity and a contrast agent, and the diagnosis by viewing the contrast agent.

The term "contrast agent" has been previously defined in the context of the uses of the invention. The term "diagnostic agent", in the context of the present invention, relates to an agent comprising a contrast agent and a compound which induces cardiotrophin-1 activity and which, administered to an individual, can be detected by contrast. The diagnostic agent includes, in addition to the compound of the invention, other components or excipients that are necessary for their use or administration.

In another aspect, the invention relates to a composition comprising a compound which induces cardiotrophin-1 activity and an antibiotic for use in the prevention and/or treatment of antibiotic-associated nephrotoxicity. In another aspect, the invention relates to the use of a composition comprising a compound which induces cardiotrophin-1 activity and an antibiotic for the preparation of a medicament for the prevention and/or treatment of antibiotic-associated nephrotoxicity. In another aspect, the invention relates to a composition comprising a compound which induces cardiotrophin-1 activity and an antibiotic for its use in the preparation of a medicament for the prevention and/or treatment of antibiotic-associated nephrotoxicity. In another aspect, the invention relates to a method of treatment or the prevention in an individual of the nephrotoxicity associated to an antibiotic comprising the administration to said individual of a composition comprising a compound which induces cardiotrophin-1 activity and an antibiotic.

The term "antibiotic" has already been defined in the context of the uses of the invention. Said antibiotics are typically used in the treatment of infections, which are diseases produced by the colonization of a host individual by a pathogenic microorganism (bacteria, fungi or protozoa) which is harmful for the normal functioning and survival of the host. The antibiotic used in the composition will determine the infection for which treatment the composition of the invention is suitable. Infections where the administration of a composition according to the invention may be useful are, without limitation, bacterial infections caused by *E.coli, Klebsiella, Pseudomonas aeruginosa, Proteus, Haemophilus influenzae, Enterobacter, Neisseria, Staphylococcus aureus, Mycoplasma, Yersinia, Plesiomonas, Aeromonas, Acinetobacter, Chlamydia, Rickettsia, Streptococcus, Corynebacterium, Bacteroides fragilis, Fusobacterium, Clostridium difficile, Clostridium perfringens, Peptostreptococcus.*

When the antibiotic is an aminoglycoside antibiotic the infections which are treated with the composition of the invention are, generally, severe infections caused by Gram negative bacteria, such as *Escherichia coli* and *Klebsiella,* as well as infections due to *Pseudomonas aeruginosa* and non-facultative anaerobic bacteria. They are also useful in surgery prophylaxis.

When the antibiotic is a cephalosporin the infections treated with the composition of the invention are, generally, infections due to Gram positive cocci, *Proteus, Escherichia coli, Klebsiella,* Gram negative bacilli, *Haemophilus influenzae, Enterobacter, Pseudomonas, Neisseria, Staphylococcus aureus.* They are also useful in the treatment of infections due to organisms resistant to other beta-lactams, such as certain presentations of meningitis, and in prophylaxis before surgery.

In another aspect, the invention relates to a composition comprising a compound which induces cardiotrophin-1 activity and an immunosuppressive agent for use in the prevention and/or treatment of immunosuppressive agent-associated nephrotoxicity.

In another aspect, the invention relates to the use of a composition comprising a compound which induces cardiotrophin-1 activity and an immunosuppressive agent for the preparation of a medicament for the prevention and/or treatment of immunosuppressive agent-associated nephrotoxicity. In another aspect, the invention relates to a composition comprising a compound which induces cardiotrophin-1 activity and an immunosuppressive agent for its use in the preparation of a medicament for the prevention and/or treatment of immunosuppressive agent-associated nephrotoxicity. In another aspect, the invention relates to a method of treatment or the prevention in an individual of the nephrotoxicity associated to an immunosuppressive agent comprising the administration to said individual of a composition comprising a compound which induces cardiotrophin-1 activity and an immunosuppressive agent.

The term "immunosuppressive agent" has already been previously defined in the context of the uses of the invention. The compositions of the invention are of use in those patients suffering from or who are susceptible to suffer from rejection in transplants or autoimmune disease and which must be treated with a nephrotoxic immunosuppressive agent. "Rejection in transplants" is understood as the reaction produced by the immune system of the recipient of a transplant which attacks the organ or tissue transplanted causing what is known as graft versus host disease and destroying the foreign tissue. "Autoimmune disease" is understood as those diseases wherein the immune system attacks the cells of the organism. Illustrative, non-limitative, examples of autoimmune diseases which may be treated with the compositions of the invention include, Addison's disease, alopecia areata, ankylosing spondylitis, haemolytic anemia, pernicious anemia, aphthous ulcers, aphthous stomatitis, arthritis, atherosclerosis, osteoarthritis, rheumatoid arthritis, autoimmune asthma, autoimmune hemolysis, Behçet's disease, Boeck's disease, intestinal inflammatory disease, Crohn's disease, choroiditis, ulcerous colitis, celiac disease, cryoglobulinemia, dermatitis herpetiformis, dermatomyositis, insulin-dependent diabetes, juvenile diabetes, autoimmune demyelinating diseases, encephalomyelitis, allergic encephalomyelitis, endophthalmia, allergic enteritis, autoimmune enteropathy syndrome, erythema nodosum leprosum, idiopathic facial paralysis, chronic fatigue syndrome, rheumatic fever, glomerulonephritis, Goodpasture syndrome, Graves syndrome, Hamman-Rich disease, Hashimoto's disease, sudden hearing loss, chronic hepatitis, Hodgkin's disease, paroxysmal hemoglobinuria, hypogonadism, regional ileitis, iritis, leukopenia, disseminated erythematosus lupus, systemic erythematosus lupus, cutaneous erythematosus lupus, lymphogranuloma, infectious mononucleosis, myasthenia gravis, transverse myelitis, idiopathic primary myxedema, nephrosis, sympathetic ophthalmia, orchitis granulomatosa, pancreatitis, pemphigus vulgaris, polyarteritis nodosa, chronic polyartritis, polymyositis, acute polyradiculitis, psoriasis, purpura, gangrenous pyoderma, Reiter's syndrome, sarcoidosis, ataxic sclerosis, progressive systemic sclerosis, scleritis, sclerodermia, multiple sclerosis, disseminated sclerosis, infertility due to antispermatozoid antibodies, thrombocytopenia, thymoma, acute anterior uveitis, vitiligo, the rejection of a transplant due to the transplant of a tissue or organ and graft versus host disease.

In another aspect, the invention relates to a composition comprising a compound which induces cardiotrophin-1 activity and an antineoplastic agent for use in the prevention and/or treatment of antineoplastic agent-associated nephrotoxicity. In another aspect, the invention relates to the use of a composition comprising a compound which induces cardiotrophin-1 activity and an antineoplastic agent for the preparation of a medicament for the prevention and/or treatment of antineoplastic agent-associated nephrotoxicity. In another aspect, the invention relates to a composition comprising a compound which induces cardiotrophin-1 activity and an antineoplastic agent for its use in the preparation of a medicament for the prevention and/or treatment of the nephrotoxicity associated to the antineoplastic agent. In another aspect, the invention relates to a method of treatment or the prevention in an individual of the nephrotoxicity associated to an antineoplastic agent comprising the administration to said individual of a composition comprising a compound which induces cardiotrophin-1 activity and an antineoplastic agent.

The term "antineoplastic agent" has already been defined in the context of the uses of the invention. The compositions of the invention are of use in those patients who suffer from or who are susceptible to suffer from cancer and which must be treated with a nephrotoxic antineoplastic agent. The term "cancer" or "tumour" is defined as the physiological condition in mammals characterized as deregulated cell growth. The compositions of the invention are useful in the treatment of any cancer or tumour, such as, without limitation, breast, heart, lung, small intestine, colon, spleen, kidney, bladder, head, neck, ovarian, prostate, brain, pancreas, skin, bone, bone marrow, blood, thymus, uterus, testicles and liver tumours. In particular, tumours which may be treated with said compositions include, without limitation, adenoma, angiosarcoma, astrocytoma, epithelial carcinoma, germinoma, glioblastoma, glioma, haemangio-endothelioma, haemangio-sarcoma, hematoma, hepatoblastoma, leukemia, lymphoma, meduloblastoma, melanoma, neuroblastoma, osteosarcoma, retinoblastoma, rhabdomyosarcoma, sarcoma and teratoma. In particular, the tumour/cancer is selected from the group of acral lentiginous melanoma, keratosis actinic adenocarcinoma, cystic adenoidal carcinoma, adenoma, adenosarcoma, adenosquamous carcinoma, astrocytic tumours, Bartholin's gland carcinoma, basal cell carcinoma, bronchial gland carcinoma, capillary carcinoid, carcinoma, carcinosarcoma, cholangiocarcinoma, cystadenoma, endodermal sinus tumour, endometrial hyperplasia, endometrial stroma sarcoma, endometrioid adenocarcinoma, ependial sarcoma, Ewing's sarcoma, focal nodular hyperplasia, germ-line tumours, glioblastoma, glucagonoma, hemangioblastoma, haemangio-endothelioma, hemangioma, hepatic adenoma, hepatic adenomatosis, hepatocellular carcinoma, insulinoma, intraepithelial neoplasia, intraepithelial squamous cell carcinoma, invasive squamous cell carcinoma, large cell carcinoma, leiomyosarcoma, melanoma, malignant melanoma, malignant mesothelial tumour, meduloblastoma, medulloepithelioma, mucoepidermoid carcinoma, neuroblastoma, neuroepithelial adenocarcinoma, nodular melanoma, osteosarcoma, serous papillary adenocarcinoma, pituitary tumours, plasmacytoma, pseudosarcoma, pulmonary blastoma, renal cell carcinoma, retinoblastoma, rhabdomyosarcoma, sarcoma, serous carcinoma, small cell carcinoma, soft tissue carcinoma, somostatin secretor tumour, squamous carcinoma, squamous cell carcinoma, undifferentiated carcinoma, uveal melanoma, verrucous carcinoma, vipoma, Wilm's tumour.

The invention is described below by the following examples, which must be considered as merely illustrative and in no case limitative of the scope of the present invention.

### EXAMPLES

### A. EFFECT OF CT-1 BLOCKADE WITH ANTI-CT-1 ANTIBODIES

### MATERIALS AND METHODS

### Reagents and chemicals

Mouse anti Cardiotrophin-1 antibody and pre-immune Goat IgG Control were provided by Digna-Biotech (Pamplona, Spain) and generated by R&D Systems (Minneapolis, MN). Both drugs were dissolved in 0.9% saline solution to a final concentration of 5 µg/ml. For anesthesia, a mixture of ketamine (Ketolar, Pfizer), benzodiazepine (Valium, Roche) and atropine (Atropina, Braun) in proportion 2:2:1 was used. For postoperative period, 0.3 mg/ml buprenorphine (Buprex, Schering-Plough) was purchased.

### Animals

Male wild-type C57BL/6 mice (Charles River Laboratories S.L., UK) were housed under standard animal care conditions and fed with standard mouse chow and water supplied *ad libitum* before and after operation. Induction of I/R injury was established using a protocol previously described (Docherty, N.G. et al. 2006. Nephrol. Dial. Transplant. (8): 2106-19). The time of ischemia chosen and the outcome were based on that found to maximize reproducibility of renal functional impairment while minimizing mortality in these animals (Chatterjee P.K. et al. 2003. Kidney Int. 63: 853-865).

### Renal ischemia/reperfusion

Animals were anaesthetized using intraperitoneal injection of anesthetic mixture (ketamine-benzodiazepine-atropine) at a dose of 60 mg/kg body weight; core body temperature was maintained at 37°C using a homeothermic blanket. Afterwards, a midline laparotomy was performed. Mice from the I/R groups were subjected to renal ischemia for 30 minutes. For this purpose, the left kidney renal artery and vein were identified and clamped using microvascular clamps. The right kidney was removed 5 minutes before the end of ischemia period (right unilateral nephrectomy). After the renal clamps were removed, the left kidney was observed for 5 additional minutes to ensure reflow, and then 1 ml of saline solution at 37°C was injected into the abdomen. Finally, the incision was sutured in two layers with running sutures using vicryl 3-0. Groups of animals pretreated were given intravenously anti-CT-1 antibody or goat preimmune IgG, through the penile vein, 15 minutes before the ischemia period. Sham-operated mice used as controls, underwent identical surgical procedures as I/R mice, with the exception that microvascular clamps were not applied (Rodríguez-Peña, A. et al. 2004. Am. J. Transplant, 4(10): 1605-1613; Garcia-Criado, F.J. et al. 1998. Transplantion, 66(8): 982-990).

### Postoperative period

After surgery mice were returned to their cages, where they were allowed to recover from anesthesia and were observed for 24 hours. Postoperative analgesia was achieved by subcutaneous injection of buprenorphine at a dose of 0.01 mg/kg body weight (Aller, M.A. et al. 2009. Liver Int. 29(8): 1132-40). Animals had free access to water and mouse chow *ad libitum.* Body weight and the physical activity were monitored during the study.

### Samples collection

In order to test kidney function, blood samples were collected as previously described (Morales, A.I. et al. 2006. Toxicol. Appl. Pharmacol. 210: 128-135). They were obtained from the tip of the tail using heparinized capillary (approximately 200 µl), both at the beginning of the surgical procedure and 24 hours after ischemia. After centrifugation of blood, the capillaries were broken by the boundary-plasma cells to proceed to the extraction of plasma that was stored in eppendorf tubes at -20°C for further analysis.

### Animal sacrifice

On the basis of preliminary and published data (Jerkic, M. et al. 2004. Nephrol. Dial. Transplant, 19:83-94; Morales, A.I. et al. 2002. Antiox. Redox Signal, 4: 893-898), the inventors have observed that this surgical procedure provokes reversible acute renal damage with a recuperation of the normal function after 7 days. As the inventors have consistently found maximal damage between 24 and 48 hours, they chose to obtain renal samples at 48 hours after I-R.

Mice were sacrificed at the indicated time points post-reperfusion, 4 hours (Groups 1-4) and 48 hours (Groups 5-8). After the reperfusion period, mice were anesthetized intraperitoneally with sodium pentobarbital (40 mg/kg body weight), blood samples were collected via cardiac puncture and mice were in *situ* perfused with cold isotonic saline solution throughout the abdominal aorta at the level of the iliac bifurcation. Blood samples were centrifuged (6000 g for 3 min) to separate plasma that was frozen and stored at -80°C for further biochemical studies. Left kidney was removed from mice at the end of the experimental period. The renal pedicle was tied and weighed. A piece of tissue was trimmed down, fixed in 4% buffered formaldehyde for 24h and embedded in paraffin for histopathological and immunohistochemical evaluation. Another portion of kidney was fast frozen by immersion in liquid nitrogen and stored at -80°C for further biochemical analysis (Sánchez-González, P.D. et al. 2010. Nephrol. Dial. Transplant, (11): 3484-95).

### Determination of plasma urea

The biochemical determination of urea concentrations in plasma samples was performed using an automated method (Reflotron Plus®; Roche Diagnostics, Barcelona, Spain) with commercial diagnostic kits (Roche Farma, Spain) as indicators ofrenal dysfunction (Grande, M.T. et al. 2010. Kidney Int., 77(6): 509-518).

### Determination of lipid peroxides (malonyldialdehyde)

Malondialdehyde (MDA) levels in kidney samples were determined using TBARs analysis as an indicator of lipid peroxidation, previously described (Rodriguez-Peña, A. et al. 2002. Hypertension, 40(5): 713-20). Tissues were homogenized in a phosphate /sodium chloride solution. An aliquot of the homogenate was added to a reaction mixture that contained 20% trichloroacetic acid and 0.67% thiobarbituric acid. The mixture then was boiled for 15 minutes at 100°C and centrifuged at 9,000 g for 5 min. The absorbance was measured in the supernatant by spectrophotometry at 532 nm.

### Determination of myeloperoxidase activity (MPO)

Myeloperoxidase (MPO) activity in kidney samples was determined as an index of polymorphonuclear cell (PMN) accumulation, using enzyme-linked immunosorbent assay (ELISA) kit specific for the previously mentioned mouse enzyme according to the manufacturer's instructions and guidelines (Hycult Biotech, The Netherlands). The concentration of MPO in kidney homogenate samples was expressed as ng/mg protein.

### Determination of Tumor Necrosis Factor-alpha (TNF-α) levels

Serum level of TNF-α was quantified using enzyme-linked immunosorbent assay (ELISA) kit specific for the previously mentioned mouse cytokine according to the manufacturer's instructions and guidelines (R&D Systems, Minneapolis, MN). The concentration of TNF-α in plasma was expressed as pg/ml.

### CT-1 western blot

Extracts from kidney tissue were prepared and analyzed by Western blot according to standard protocols (Suzuki, Y. et al. 2012. Biotech. Histochem., 87(4): 241-8). Briefly, extract samples that contained 100 µg proteins were separated by 10% SDS-PAGE gel electrophoresis and transferred to PVDF membranes. After being blocked with 5 % BSA, the membranes were incubated at 4°C overnight with the anti-CT-1 antibody (dilution 1:500; R&D Systems). Then, previously to three washes with TBST, membranes were incubated with secondary HRP-conjugated anti-mouse IgG for 1 hour (dilution 1:10,000; Bio-Rad Laboratories). After washing, the immune complexes were detected with chemiluminiscent (ECL) HRP substrate using an image reader (ImageQuant RT ECL, GE Healthcare, Spain), and the bands were measured. Membranes were also reprobed with mouse monoclonal anti-GADPH antibody (1:20,000; Ambion Applied Biosystems) to verify equal loading of protein in each lane.

### Histopathological examinations

Formaldehyde-fixed tissue samples were dehydrated in an ascending series of ethanol (60, 80, 96, 100%), rinsed with toluene and cleared in xylene. Then, each sample was included in melted paraffin at 60°C, forming blocks in which the piece is properly oriented tissue. With the help of a microtome, 4 µm sections were cut and deposited on glass slides (Morales, A.I. et al. 2006. Food Chem. Toxicol., 44(12): 2092-100).

### Haematoxylin & eosin staining

Paraffin-fixed kidney sections were mounted on glass slides and stained with H&E for being examined with light microscopy. Five randomly selected images per section were digitally captured (magnification × 400) and the optical density was analyzed (Grande, M.T. et al. 2010. Kidney Int., 77(6): 509-518).

### Immunohistochemical staining of CT-1 and ED-1

Sections were treated with trypsin for ED-1 staining (CD-68), or microwave for cardiotrophin-1 staining. Renal tissue was then incubated with monoclonal anti-ED-1 antibody (1:200) (M0814; DakoCytomation, Denmark) and polyclonal anti-CT-1 antibody (1:200) (AF438; R&D Systems, Minneapolis, MN). Incubation with primary antibodies was carried out overnight at 4°C in a humidified chamber, followed by a second reaction with antibody (1:100, Cy3-conjugated rabbit anti-rat, Jackson Laboratories, Germany). Nuclear DNA was counterstained with DAPI and immunofluorescence staining was visualized using a Laser Scanning Spectral Confocal Microscope (TCS SP2, Leica) at magnification of x 400 (Grande, M.T. et al. 2010. Kidney Int., 77(6): 509-518).

### Data statistical analysis

Statistical analysis was performed using the NCSS software. Values were expressed as mean of n independent experiments. Values from data with a normal distribution were expressed as mean ± standard error of the mean (SEM). Scheffe's correction test was used for multiple comparisons. For data not conforming to a normal distribution, values were expressed as median and the Kruskal-Wallis test was used for multiple comparisons. In general, maximum alpha error assumed and considered statistically significant in all previous tests was p <0.05 or Z>1.96.

### 1. Effect of CT-1 blockade with anti-CT-1 antibodies on the severity of acute renal failure induced by unilateral renal ischemia

In this study the effect of CT-1 blockade with anti-CT-1 antibodies on the severity of acute renal injury induced in a mouse model of unilateral renal ischemia was investigated.

Mice were housed 4-5/cage and kept at the animal house facility with room temperature of 20 ± 2°C, 50% humidity and 12:12h of light-dark cycles. They were fed with a standard pellet diet, and administered tap water *ad libitum.* After a 7-day acclimatization period, a total of 60 mice were randomly distributed into the following eight groups:
- GROUP 1. Control 4h group (Control-4h, n = 4); mice that received a single dose bolus of saline solution 15 minutes before renal ischemia (30 minutes), followed by uninephrectomy and reperfusion for 4 hours.
- GROUP 2. Control IgG 4h group (Control IgG-4h, n = 8); mice that received a single dose bolus of goat preimmune IgG 15 minutes before renal ischemia (30 minutes), followed by uninephrectomy and reperfusion for 4 hours.
- GROUP 3. Anti-CT-1 4h group (Anti-CT-1-4h, n = 8); mice that received a single dose bolus of anti-CT-1 antibody (50 µg/kg, i.v.) 15 minutes before renal ischemia (30 minutes), followed by uninephrectomy and reperfusion for 4 hours.
- GROUP 4. Sham 4h group (Sham-4h, n = 4); mice that received a single dose bolus of saline solution 15 minutes before surgical procedures described above, with the exception that renal I/R were not applied.
- GROUP 5. Control group (Control, n = 10); mice that received a single dose bolus of saline solution 15 minutes before renal ischemia (30 minutes), followed by uninephrectomy and reperfusion for 48 hours.
- GROUP 6. Control IgG group (Control IgG, n = 10); mice that received a single dose bolus of goat preimmune IgG 15 minutes before renal ischemia (30 minutes), followed by uninephrectomy and reperfusion for 48 hours.
- GROUP 7. Anti-CT-1 group (Anti-CT-1, n = 10); mice that received a single dose bolus of anti-CT-1 antibody (50 µg/kg, i.v.) 15 minutes before renal ischemia (30 minutes), followed by uninephrectomy and reperfusion for 48 hours.
- GROUP 8. Sham group (Sham, n = 6); mice that received a single dose bolus of saline solution 15 minutes before surgical procedures described above, followed by uninephrectomy and reperfusion for 48 hours.

### Effect of anti-CT-1 antibody on clinical aspects and body weight

Figure 34 represents the body weight of animals in the four experimental groups at 48 hours post-I/R during the study period. All the mice in sham group showed a good aspect, normal motility and a stable maintenance in body weigh at all time points. However, I/R groups showed a poor aspect and lower motility accompanied by a significant loss weight after reperfusion phase. This health deterioration caused by surgical procedure was slightly greater in anti-CT-1-treated mice as compared with that of control groups (saline or goat IgG treated-mice).

### Effect of anti-CT-1 antibody on the survival rate

Figure 35 represents the survival rate of mice in the four experimental groups at 48 hours post-I/R during the experimental period. All the mice without surgical procedure (sham group) presented a normal survival and no mortality was observed in this group. However, animals with I/R presented a slight mortality with a survival of about 90% (9 out of 10 animals) within 24 hours post-ischemia. The survival rate decreased to 70% (7 out of 10 animals) in the anti-CT-1 group and 80% (8 out of 10 animals) in the control groups (saline or goat IgG treated-mice) at 48 hours post-ischemia.

### Effect of anti-CT-1 antibody on renal function

Figure 36 represents the plasma urea levels evolution in the four experimental groups at 48 hours post-I/R during the study. The results show that plasma urea concentration remained unchanged in sham group. Regarding mice subjected to renal I/R, plasma urea levels increased at 24 hours post-ischemia, reaching a maximum peak. This increase in renal dysfunction was higher in anti-CT-1-treated mice than in control groups (saline or goat IgG-treated mice), but there were no significant differences among these groups. At final time points or 48 hours post-ischemia, plasma urea concentration returned to almost normal levels in all I/R groups.

### Effect of anti-CT-1 antibody on renal inflammation

Figure 37 represents renal malondialdehyde (MDA) levels in the four experimental groups at 48 hours post-I/R. Compared with sham group, the kidneys from mice subjected to I/R demonstrated an increase in MDA levels, suggesting an increased lipid peroxidation in renal tissues. Tissue levels of MDA observed in anti-CT-1-treated mice were significantly higher than those observed in the saline control.

Figure 38 represents the renal myeloperoxidase (MPO) levels in the four experimental groups at 48 hours post-I/R. Kidneys from mice that were subjected to I/R demonstrated a significant increase in MPO levels compared with sham-operated mice, suggesting an increased PMN infiltration into renal tissues. Kidneys of I/R anti-CT-1-treated mice had a significant higher level of MPO activity than kidneys from control and control IgG groups. No significant differences in renal MPO levels were found between renal tissue of control and control-IgG mice.

Figure 39 represents the plasma levels of TNF-α in the four experimental groups at 4 hours post-I/R. I/R caused significant elevation of TNF-α levels in serum compared with those of sham group. Anti-CT-1 therapy caused non-significant changes in serum TNF-α levels compared with I/R control and control IgG groups.

### Effect of anti-CT-1 antibody on renal CT-1 expression

Figure 40 represents renal expression of CT-1, assessed by Western blot in the four experimental groups at 48 hours after I/R. The expression of CT-1 was increased in mice in all I/R groups compared with sham group. The treatment with anti-CT-1 antibody, resulted in a significant increase of this protein expression when compared with the other groups.

### Effect of anti-CT-1 antibody on renal histology

Sections of left kidneys were stained with haematoxylin-eosin and analyzed by light microscopy after 48 hours after I/R. Histologic examination demonstrated normal renal architecture in sham-operated mice, but those that underwent I/R showed a significant degree of renal injury. Specifically, kidneys exhibited degeneration of tubular structure, tubular dilatation, swelling, tubular cell necrosis, and luminal congestion. Inflammatory infiltrate was visible in perivascular regions. In anti-CT-1 group, slice sections of kidneys showed a marked increase in the severity of these histologic features of renal injury when compared with kidneys subjected to I/R only. There were no differences in any of the above histologic parameters measured between control and control-IgG mice (data not shown).

### Effects of anti-CT-1 antibody on renal macrophage infiltration

Sections of left kidneys obtained 48 hours after I/R were stained with a macrophage-specific marker (anti-CD68 antibody). Two days after I/R (48 hours post-ischemia), CD-68 positive cells (ED-1 positive) were predominately expressed in the interstitium around atrophic tubules. CD68-positive cell number post-I/R was significantly higher in anti-CT-1-administered mice than in control groups. No significant differences in macrophage infiltration were found between control and control-IgG mice. Macrophage staining was minimal in kidneys of sham group (data not shown).

### B. THERAPEUTIC EFFECTS OF CT-1

### MATERIALS AND METHODS

### PREPARATION AND ANALYSIS OF THE SAMPLES

### Obtainment of urine.

The rats were placed in individual metabolic cages where they had free access to food and drink. After two days of acclimatization, the 24-hour urine was collected in graduated cylinders. The urine collected was centrifuged during 8 minutes at 2,500 rpm, to clean it from residues not thereof, the clean urine was collected and it was stored at - 80° C for its later analysis.

### Obtainment of blood and plasma.

The blood samples were obtained in capillaries with heparin by a small cut at the end of the tail. Five capillaries were extracted per rat (200 µL), and the capillaries were centrifuged in a microcentrifuge at 11,000 rpm during three minutes. Once centrifuged, the capillaries were broken with the aid of a file, by the cells-plasma limit. The plasma was extracted and stored in eppendorf tubes at -80° C for its later analysis.

### Extraction and processing of the kidneys for histology and Western blot.

At the end of each experiment, the animals were sacrificed and the kidneys were extracted for their *post mortem* study. Before the extraction, the kidneys were perfused with heparized saline solution to remove the blood from its vascular tree. For this, the rats were anaesthetized intraperitoneally with pentobarbital sodium with a dose of 10 mg/kg of weight. To access the abdominal cavity, an incision was made in the abdomen (laparotomy) and the intestinal mass was separated to cannulate the aorta in the iliac bifurcation. Through the cannula, 20 mL of heparized saline solution were perfused at 37° C. After this, the kidneys were decapsulated, extracted and sagitally sectioned in two halves. One kidney and the half of the other were instantly frozen by immersion in liquid nitrogen and stored at -80°C to later perform *Western blot* studies. The other half was introduced in 4% formaldehyde buffered at pH 7 and stabilized with methanol, for histology studies. Once fixed in formaldehyde, after remaining in this solution for 24 hours, the kidneys were dehydrated by passing through a battery of alcohols of increasing graduation, starting with ethanol. Later, each sample was included in 60° melted paraffin in an oven for 1 hour, forming blocks wherein the piece is suitably oriented. 3 µm thick sections were cut with a microtome (MICRON HM-310) and then the cuts were placed on a glass slide. The cuts were deparaffinised with xylene and the sample was rehydrated making it pass through a series of decreasing ethyl alcohol concentrations until reaching 100% distilled water.

### Preparation of the tissue homogenate for Western blot.

The kidney samples cold stored at -80°C, were pulverized by shaking between two metal plates, maintaining them at all time in dry ice to avoid their thawing and deterioration. Then, 100 mg of tissue powder of each of the samples was weighed, whereby a tissue extract was made. For this, they were homogenized with approximately 400 mL of lysis buffer (140 mM NaCl, 50 mM EDTA, 10% glycerol, 1 % NP-40, 20 mM Tris pH=7.5, 1mg/ml leupeptin 1mg/ml aprotinin, 5mmol/l phenyl methyl sulfonyl fluoride (PMSF), 5mM NaF and 1mmol/l pH=7.5 Na₃VO₄) for each 40 mg of tissue powder, and with the aid of a titrator (Politron), the samples were homogenized. Then, the tissue lysate was centrifuged during 20 minutes at 14,000 g at 4°C. Finally the supernatant was collected and it was frozen at -80°C until the time of its use. One aliquot was used to determine the total proteins of the samples using a commercial kit based on Lowry's colorimetric method (Lowry et al. J Biol Chem.1951; 193:265).

### ANALYSIS OF KIDNEY FUNCTION

### Determination of creatinine and creatinine clearance

To measure kidney function, the plasma concentrations of creatinine and creatinine clearance, which is a measurement of the glomerular filtration rate, were determined. The creatinine clearance (CrCl) was calculated from the formula CrCl = UF x CrU/CrP, where UF is the urinary flow (ml/min), CrU the creatinine concentration in urine (mg/ml) and CrP the creatinine concentration in plasma (mg/ml). The creatinine concentration in urine and in plasma were measured by a semiautomatic analyser (Reflotron, Roche).

### Determination of plasma urea

The concentration of urea in plasma was measured by specific reactive strips of a commercial kit, using a semiautomatic analyser (Reflotron, Roche).

### Determination of proteinuria

The concentration of proteins in urine (proteinuria) was measured by Bradford's colorimetric method (Anal Biochem. 1976; 72:248-54). The urinary excretion of proteins (UEₚᵣₒₜ) in 24 hours was calculated by the formula: **UEₚᵣₒₜ = Cₚᵣₒₜ x FU_{24 h}**, where Cₚᵣₒₜ is the concentration of proteins in urine (mg/mL) and FU_{24 h} the urinary flow of 24 hours (mL/day).

### Determination of the urinary flow

The urinary flow (mL/day) was evaluated measuring the graduated receptacles containing the 24-hour urine in the metabolic cages as indicated in the obtainment of urine.

### Determination of N-acetyl glucosaminidase (NAG)

For its determination a commercial kit from Roche was used, following the manufacturer's specifications, and the results were analysed with the *KC-Junior* software.

### Determinations by Western blot of KIM-1 and NGAL.

In the Western Blot technique, 100µg of protein of the total of the renal tissue homogenate or a volume of urine of each animal which corresponds to the excretion fraction were loaded in a polyacrylamide gel. After separation by electrophoresis, these proteins were transferred to a PVDF membrane (Inmobilon-P #IPVH00010, Madrid, Spain), and they were hybridized with antibodies against KIM-1 (R&D Systems) and NGAL (MBL).

### Determination of lipid peroxides (malonyldialdehyde)

The malonyldialdehyde was determined from a spectrophotometric technique based on the reaction of two molecules ofthiobarbituric acid (TBA) with a molecule of MDA (end product of lipid peroxidation) at 45°C which leads to a stable chromophore with a maximum absorbance at 532nm.(Recknagel RO, Ghoshal AL, 1966, Exp Mol Pathol 5:413-426).

The tissue samples were homogenized with a polytron in homogenate solution (20mM phosphate / 50mM NaCl, pH =7.4) in 1:5 proportion (w:v; mg/µL) with 70% perchloric 1: 0.05 (w:v; mg/µL). It was centrifuged at 4,200 rpm during 15 minutes. The supernatant was taken for the assay and to determine proteins using the Lowry method. 200µL of the supernatant were loaded in a 96-well plate. The reaction was performed and the absorbance was measured in the spectrophotometer at 532 nm. The results were expressed in micromoles per litre (µmol/L).

### Histological studies

### Staining with hematoxylin-eosin.

This technique reveals the morphological structure of the tissue cells, since it stains the nucleus, the acid areas of the cytoplasm and the matrix blue, and the cytoplasm and all its basic components pink. The renal histological cuts, prepared as indicated, were kept for four minutes in a hematoxylin solution and they were then washed with running water to maintain them for three minutes in an eosin solution. Once stained, the samples were again dehydrated by alcohols with increasing concentration (70°, 90°, 100°) to permanently fix them with a drop of Canada Balsam whereon a slide cover was placed with mounting medium (*Tissue-Tec*). The images were captured with a high-resolution video camera connected to an optical microscope with a 10X lens. A green optical filter was used to increase the contrast.

### Immunohistochemical studies.

The immunohistochemical technique was started by eliminating the endogenous signal of the cuts, with hydrogen peroxide and 3% methanol. Then, the cuts were blocked, and later incubated with primary and secondary antibodies. Then HRP (horseradish peroxidase) was added to the samples. Immediately after, the chromogen 3,3'-diaminobenzidine (DAB) was added. The preparations were lightly immersed in hematoxylin to contrast. Finally, the cuts were dehydrated by a battery of alcohols of increasing concentration, whereon a slide cover was placed with mounting medium (*Tissue-Tec*) so that they could be observed in an optical microscope.

### Determination of the production of the superoxide anion O₂⁻ (SOA)

To determine the SOA production levels in the kidney a technique, which is a modification of that described by Boveris for mitochondria, was followed [Boveris A. Methods Enzymol. 1984; 105: 429-435], based on the reduction of the cytochrome C by the O₂⁻ radical. Briefly:
- a cuvette for spectrometry (1 ml cuvettes) was prepared adding 100 µl of cytochrome C (75 µM), 20 µl of superoxide dismutase (SOD; approximately 264 U), 25 µl of sample, and buffer solution (0.1 M potassium phosphate + 0.1mM EDTA; pH 7.8) until completing a volume of 1,000 µl;
- a reference cuvette was prepared, without added sample, adding 100 µl of cytochrome C (75 µM), 20 µl of SOD, and buffer solution until completing a volume of 1,000 µl;
- another similar cuvette was prepared not containing SOD to study the non-specific reduction of cytochrome C;
- the reduction of cytochrome C was followed by spectrophotometric readings [at 550 nm of λ; pH 7.8 and temperature of 25 °C during 1 minute with intervals every 6 seconds, in 1 ml cuvettes, with lightpath of 1 cm] in 2 phases 1.- reduction of the non-specific cytochrome C (without SOD), and 2.- reduction of the cytochrome C dependent on the superoxide (with SOD);
- the increase in absorbance units in the reaction mixture was converted into nmoles of SOA with the molar extinction coefficient: ΔE₅₅₀ / 21.0 x 10³ M⁻¹ cm⁻¹; considering the case that the cytochrome C in the reference cuvette is totally oxidised and that the increase in absorbance observed solely represents the absorbance of the reduced product, Δ absorbance: reduced - oxidised;
- In this way, the production of the superoxide anion SOA was expressed in nmol / mg protein / minute.

### Determination of myeloperoxidase activity (MPO)

The presence of myeloperoxidase, a specific enzyme of neutrophils used as index to assess the neutrophilic infiltration in the kidney, was analysed in the kidney samples by the following method (Mullane KM et al, J Pharmacol Methods 1985, 14(3):157-167): once obtained, the samples were weighed among ice and frozen in liquid nitrogen later being stored at -80° C until their determination. Later, the samples were homogenized in a solution composed of a 50 mM potassium phosphate buffer with 0.5% hexadecyltrimethylammonium bromide and 0.146% EDTA at a pH of 6.0 and in the proportion of one gram of tissue per 10 ml of homogenized solution. Then they were homogenized and subjected to a sonication process among ice 10 times and five seconds each, in this way to break the cells, among them the neutrophils, and leave the myeloperoxidase free in the solution. This homogenate was centrifuged for 30 minutes at 15,000 g maintaining the temperature inside the centrifuge between 3 and 4° C. The supernatant was decanted and incubated during 2 hours at 50° C to eliminate other types of peroxidase and other compounds that interfere in determination of the myeloperoxidase. The buffer was prepared for the assay composed of 50 mM potassium phosphate buffer with 0.167 mg/ml of O-dianisidine dichloride and 0.005% hydrogen peroxide at a pH of 6.0. Against a blank with the assay buffer, the standard curve was performed with known quantities of MPO at 460 nm wavelength (λ) and at 25° C. The unit (U) of activity of the myeloperoxidase was defined as the quantity of enzyme that degrades 1 µmol of H₂O₂ / minute at 25° C.

### Determination of TNFα, IL-1β, IFN-γ, IL-6 and IL-10 cytokines

The levels of these cytokines in plasma were determined by ELISA using DuoSet ELISA Development System rat kit in accordance with the manufacturer's instructions. In all cases the levels were expressed in pg/ml. Principles of the assay: It is an ELISA kit which uses multiple antibodies with the "sandwich" principle. In first place, a 96-well plate was used with an anticytokine monoclonal antibody adhered on each of them to capture the cytokine present in the samples and standards that were added in duplicate in each of the wells together with the corresponding blanks. After washing the dish to eliminate the non-adhered material, an anti-cytokine polyclonal conjugated peroxidase was added. Then the plate was washed again to eliminate the non-adhered material and the substrate solution was added which started the peroxidase catalysation. The colour change was stopped by acidification.

The absorbance was measured at 450 nm λ the results obtained being proportional to the quantities of cytokines of the samples, which were calculated by interpolation with the standard curve.

### 1. Study of the effect of cardiotrophin-1 in contrast-induced nephropathy

An experimental model of iodinated contrast nephropathy was used, consisting of the intravenous administration of the iodinated contrast Gastrografin (Bayer C.N. 909622 EXO) to rats predisposed to suffering acute kidney failure. The predisposition was achieved by the prior treatment with gentamicin at subtoxic doses (Quirós et al., Kidney Int. 2010; 78: 1006-1015).

The experiment was performed on 32 male Wistar rats of around 220 grams in weight. The environmental conditions of the animals were kept constant: temperature of around 20°C, humidity of approximately 60% and daily light period (photoperiod) of 12 hours.

Six experimental groups were considered in the design study:
- Control group (C): rats administered saline solution (n= 5).
- Gentamicin group (G): rats administered gentamicin i.p. (50 mg/kg/day) during 6 days, starting 6 days before the administration of the contrast. Previous studies of our laboratory have demonstrated that this treatment does not cause any detectable change in the kidney function nor tubular lesion even using the finest markers existing to date (Quirós et al, 2010) (n= 5).
- Cardiotrophin group (CT-1): rats administered cardiotrophin i.v. (100 µg/Kg/day) the day before the administration of the contrast and in the four subsequent days (n= 5).
- Gastrografin group (Gg): rats administered a single dose of iodinated agent i.v. (10 mL/Kg; 3.7 mg/Kg) (n= 5).
- Gastrografin + Gentamicin Group (Gg+G): rats administered the iodinated agent on the day following the end of treatment with gentamicin (n= 6).
- Gastrografin + Gentamicin + Cardiotrophin group (Gg+G+CT-1): rats administered the iodinated agent, gentamicin and cardiotrophin (n= 6).

The animals were housed in metabolic cages in order to collect the urine free of faeces. Likewise, a blood sample was obtained every day (0.150 ml) from the tail in heparized capillaries.

At the end of the experiments (four days after administration of the iodinated contrast) the rats were anaesthetized and, later, they were perfused with heparinized saline solution. The kidneys were obtained from these animals and part of the samples was introduced in formol to perform histological studies whilst another part was ultrafrozen for the tissue studies.

The studies performed were the following:
- Plasma creatinine, Plasma urea, proteinuria, microalbuminuria
- Creatinine clearance in metabolic cages
- Glomerular filtration rate (GFR; inulin clearance), renal plasma flow (RPF; para-aminohippuric acid clearance), renal vascular resistance (RVR).
- nephrotoxicity urinary markers: NAG (N-acetyl glucosaminidase), LDH (lactate dehydrogenase), GGT (gamma glutamyltransferase), KIM-1 (Kidney Injury Molecule 1), PAI-1 (plasminogen activator inhibitor 1), Vimentin.

### Survival

In groups C, G-, Gg and CT-1 all animals survived until the end of the experiment. In groups Gg+G- and Gg+G+CT-1, one animal from each group died.

### General condition and evolution of the body weight

The animals from groups C, G- and CT-1 showed a healthy appearance and normal mobility throughout the experiment. However, the animals treated with Gastrografin showed a more deteriorated appearance and distended legs, which recovered in 15-20 minutes after its administration. Despite this, there were no important differences in the weight increases between the experimental groups (Figure 1).

### Kidney function

Group Gg+G produced a decrease in creatinine clearance (as an index of the state of glomerular filtration), which was translated into a decrease in excretion and, therefore, an accumulation in the blood of metabolism products such as creatinine, urea and their derivatives (measured as a whole as urea plasma concentration) (Figures 2-4). Likewise, the joint administration of Gg+G was accompanied by a high proteinuria (Figure 5).

The administration of CT-1 (group Gg+G+CT-1) partially reversed the decrease in creatinine clearance, the increase in plasma concentration of creatinine, urea and proteinuria (Figures 2-5). The administration of gentamicin, gastrografin and CT-1 alone did not alter any of these parameters (Figures 2-5).

### Urinary markers of nephrotoxicity

NAG is a lysosomal enzyme which is used in the diagnosis and monitoring of nephropathies. Its presence in the urine increases as a consequence of kidney injury and is considered a marker of tubular damage. Our results show that NAG increased in animals treated with Gg+G. CT-1 reduced this increase similarly to proteinuria, which corroborates the findings related to tubular damage (Figure 6).

KIM-1 is a type I membrane protein, which has been developed as an early marker more sensitive than NAG. During tubular damage, the extracellular domain of KIM-1 is split and it is detected increased in the urine as a very early marker of damage. In figure 7 it can be observed how the joint treatment of Gg+G induces a clear increase in the reactive bands to the anti-KIM-1 antibody in the urine. Gentamicin, gastrografin and CT-1 produced no effect in comparison with the controls. For its part, CT-1 considerably decreases the increase in KIM-1 expression produced by the joint treatment of Gg+G.

Plasminogen activator inhibitor 1 (PAI-1) is an inhibitor of the fibrinolysis process, which is involved in inflammatory responses and tissue repair. Its expression is increased in many cell types as a consequence of the action of inflammation mediators. It also increases as a consequence of chronic and acute kidney injury. Our results indicate that PAI-1 expression is clearly increased in the urine of animals administered the contrast and previously sensitized (Gg+G group). These results suggested that in this experimental group a lesion was produced wherein inflammatory processes seem to be involved, as a repair response to damage. The levels of this marker in the rats treated with gentamicin, gastrografin or CT-1 are not different from the control group animals. The marker does not appear either in group Gg+G+CT-1, which reveals a protective effect of CT-1 (Figure 7).

### Acute kidney clearance

In the results obtained in inulin clearance a decrease is demonstrated in glomerular filtration rate (GFR) in the group treated with gentamicin and the iodinated contrast (Gg+G), which is reversed with CT-1 (Figure 8). Renal plasma flow (RPF) follows a similar pattern: decrease in the groups treated with gentamicin and the contrast, and reversal in the co-treatment with CT-1 (figure 9). Furthermore, the administration of gentamicin and gastrografin increased renal vascular resistances (RVR) (Figure 10), which could suggest the involvement of the release of vasoconstrictor factors (or the inhibition of the effect of vasodilator factors) which would hinder the passage of blood through the kidney, information already described for this type of agents. The coadministration of CT-1 with gentamicin and gastrografin counteracted the increase in RVR.

### 2. Study of the effect of cardiotrophin-1 in acute kidney injury produced by cisplatin

The experiment was performed in 24 male Wistar rats of around 220 grams in weight. The environmental conditions of the animals were kept constant: temperature of around 20°C, humidity of approximately 60% and daily light period (photoperiod) of 12 hours.

All processes related to the animals and their care have been governed in accordance with the institutional guidelines of the University of Salamanca, and in compliance with Spanish (Act 121/000123/2007/Spain; RD 1201/2005/CyL) and international (Directive 2003/65/EC) laws.

The animals were divided in 4 experimental groups, comprising 6 rats per group. Daily treatment during 7 days with cardiotrophin-1 was started one day before the sole treatment with cisplatin. The experimental design was developed as described below:
- **Group I** (*Control, C, n=6 animals*) animals treated with vehicle via i.v. during 7 days.
- **Group II** (*Cardiotrophin-1, CT-1, n=6 animals*) animals administered a daily dose of cardiotrophin-1 of 100 µg/kg body weight via i.v. during 7 days.
- **Group III** (*Cisplatin, P, n=6 animals*) animals that received a single dose of cisplatin of 6 mg/kg body weight via i.p.
- **Group IV** (*Cardiotrophin-1-Cisplatin, CT1-P, n=6 animals*) animals treated with cardiotrophin-1 and cisplatin at the same dose and administration routes as groups II and III respectively.

### Survival

The survival of the animals represents a significant data in the evolution of a toxicological study. Figure 11 represents the survival of the animals in the four study groups during the experimental period.

Treatment with cardiotrophin-1, cisplatin at a dose of 6 mg/kg and cisplatin-cardiotrophin does not modify the survival of the animals throughout the experimental period, with respect to the control group (Figure 11).

### General condition and body weight

The general condition, as well as the body weight of the animals, is an important aspect in the visual assessment of the effect of the drugs. Figure 12 represents the body weight of the animals in the four study groups during the experimental period.

The antineoplastic treatment with cisplatin at a dose of 6 mg/kg induces a significant decrease in the body weight of the animals, this reduction being significantly less in the group of animals jointly treated with cardiotrophin-1. This fact was corroborated with the external evaluation of the physical condition of the animals, wherein a substantial deterioration was observed as a consequence of the antitumoral treatment and a spectacular improvement during the coadministration of cardiotrophin-1. For its part, the cardiotrophin-1 group reveals an increase in body weight throughout the study period, without apparent differences with respect to the control group (Figure 12).

### Urinary flow

The evolution of the urinary flow represents additional and even revealing information of the kidney injury produced by a pharmacological treatment. Figure 13 represents the evolution of the urinary flow of the animals subject to study in the four experimental treatment groups. The cytostatic treatment with cisplatin at a dose of 6 mg/kg induces a mild biphasic polyuria. However, the joint administration of cardiotrophin-1 and cisplatin shows a constant evolution in the urinary flow during the experimental period, without significant differences with respect to the control and cardiotrophin-1 groups. This fact reveals a clear nephroprotection of cardiotrophin-1 in cisplatin nephropathy (Figure 13).

### Basic kidney function

The determination of plasma creatinine, as a safe and effective parameter of kidney functionality, represents a characteristic marker of drug nephrotoxicity. Figure 14 represents the evolution of plasma creatinine in the four experimental groups of animals during the study period. The antineoplastic treatment with cisplatin at a dose of 6 mg/kg induces a marked increase in plasma creatinine, mainly from day 2 post-cytostatic treatment, this increase being significantly less in the group of animals jointly treated with cardiotrophin-1. This fact determines a reversible acute kidney injury by cisplatin, wherein the intravenous treatment of 100 µg/kg of cardiotrophin-1 during 7 consecutive days is capable of protecting and reversing the nephrotoxicity induced by this antitumoral drug. For its part, the cardiotrophin-1 group does not reflect changes in said kidney parameter assessed, with respect to the control group (Figure 14).

### Weight of the organs

The evaluation of the weight of different organs constitutes complementary information to the study of the toxic activity of a drug. Figure 15 reflects the weight of different organs (with respect to the body weight of the animal) in the four study groups, at the end of the experimental period.

Antitumoral treatment with cisplatin at a dose of 6 mg/kg produces a significant increase in the final kidney weight, this increase being substantially less in the group of animals treated jointly with cardiotrophin-1 (Figure 15, upper left panel). This fact again corroborates the kidney protection exercised by the treatment with cardiotrophin-1 in acute toxic damage by cisplatin. However, the final weight of the other organs assessed (heart, lungs and liver) do not show significant changes with respect to the control group, supporting the theory of nephrotoxicity characteristic of cisplatin and the protective effect of cardiotrophin-1. For its part, the cardiotrophin-1 group does not show changes in the evolution of the body weight, with respect to the control group (Figure 15).

### 3. Study of the effect of cardiotrophin-1 on acute kidney injury produced by gentamicin

Experimental groups:
a) Control (C): vehicle once per day i.v. (n=6).
b) CT-1: CT-1 (100 µg/kg/day, i.v.), during 7 days. (n=6).
c) G: gentamicin (150 mg/kg/day, i.p.) during 6 days. (n=6).
d) G+CT-1: CT-1 (100 µg/kg/day, i.v.), during 7 days and, from day two, gentamicin simultaneously (150 mg/kg/day, i.p.) during 6 days. (n=6).

### Survival

In the control (C) and CT-1 groups, all animals survived until the end of the experiment. In groups G and G+CT-1, one animal from each group died.

### General condition and evolution of body weight

The animals from the control (C) and CT-1 groups showed a healthy appearance and normal mobility throughout the experiment. However, the animals treated with gentamicin showed a more deteriorated appearance, reduced mobility and slight colouring of the hair. These symptoms were significantly reduced in the animals from group G + CT-1. As observed in figure 16, the body weight of the rats that received G showed a slight decrease with respect to that of the control and CT-1 groups. CT-1 partially mitigated the weight loss produced by G.

### Blood pressure and heart rate

In the control (C) and G groups, the systolic blood pressure (SBP) slightly increased as a consequence of the experimentation. This slight increase was not observed in the groups that received CT-1 (Figure 17, upper panel). The heart rate very slightly increased equally in all groups throughout the experiment (Figure 17, lower panel).

### Weight of the organs

At the time of sacrifice, the weight of the right kidney and the heart (with respect to the body weight) had not been modified as a consequence of the treatments. In the case of the liver, CT-1 induced a certain hepatomegaly which disappeared in the group cotreated with G (G + CT-1). G per se, also showed a tendency for relative weight reduction of the liver which counteracted the hypertrophic effect of CT-1. However, CT-1 induced a marked splenomegaly which was not modified by cotreatment with G (Figure 18).

### Kidney function

G produced a clear acute kidney failure which was revealed by an accumulation in the blood of metabolism products such as urea and its derivatives (measured jointly as urea plasma concentration) and creatinine (Figures 19 and 20) and a drastic decrease in creatinine clearance (as a measurement of the glomerular filtration rate) (Figure 21). Likewise, G produced high proteinuria (Figure 22) which, in accordance with the specialized literature on the nephrotoxicity of this antibiotic, is of tubular origin, and results from the damage and functional alterations of this compartment. The administration of CT-1 together with G partially reversed the decrease in creatinine clearance, the increase in plasma creatinine and urea concentration, and proteinuria (Figures 19-22). CT-1, administered alone, did not alter any of these parameters (Figures 19-22).

Neither G nor CT-1, administered separately, modified the urinary flow with respect to the animals of the control group (C). However, the joint administration of these two compounds produced a synergic effect which progressively increased the urinary flow, until reaching values 3-4 times greater than the other groups (Figure 23).

### Renal tissue integrity

The condition of the renal tissue as a consequence of the action of G and CT-1 was determined by histological studies of renal sections, and by the determination of tissue and urinary markers associated to the processes of tissue damage and repair. As shown in Figure 24, which has images representing the renal cortex, gentamicin causes a massive tubular necrosis in the cortical area of the kidney, wherein many completely necrotized and other partially necrotized tubules are observed, in coincidence with the knowledge on the renal effect of this drug. Cotreatment with CT-1 moderately and partially prevents the extension of tubular damage, observing less tubules completely and partially necrotized. However, even with cotreatment with CT-1, gentamicin produces marked tubular damage. These results are, in general terms, coincident with the biochemical analysis of kidney damage and repair markers. Thus, as observed in Figure 25, the urinary excretion of N-acetyl glucosaminidase (NAG), a lysosomal enzyme which marks, among other things, tubular damage, is highly increased in the animals treated with G, in accordance with the intense damage it produces in that compartment. CT-1 reduces this increase in NAG excretion, especially the submaximal which is produced during the first days of the damage. The profile of the effect of CT-1 on NAG excretion is almost identical to that of proteinuria, which reinforces the results related to tubular damage.

Figure 26 shows how treatment with G induces a clear increase in the renal levels of KIM-1, as well as of reactive bands to the anti KIM-1 antibody in the urine. CT-1 does not produce any effect in comparison with the controls. However, it can be observed that CT-1 does not appreciably decrease the increase in KIM-1 expression in the kidney that G produces. This could indicate, among other things, that the regeneration process continues as active as in the animals treated with G. However, it is observed that the upper band (approx. 90 kDa) of KIM-1 in the urine partially decreases, which would be congruent with a partial reduction of damage.

NGAL ("*Neutrophil Gelatinase-Associated Lipocalin* ") is a lipocalin related to inflammatory processes. It has been detected increased in various forms of kidney injury, both in the tissue and in the urine, and it has been proposed as one of the earliest and most sensitive markers of kidney injury. In Figure 26 it can be observed how both G and CT-1 induce an increase of renal and urinary NGAL.

### 4. Study of the effect of cardiotrophin-1 in acute kidney injury produced by renal

### ischemia/reperfusion

Renal ischemia/reperfusion (I/R) is a typical cause of acute kidney failure and in kidney transplant it may cause acute tubular necrosis or initial delay in the function of the graft or delayed renal function (DRF). The I/R has been correlated with the incidence of acute rejection (Matas AJ. et al. Transplantation. 2000; 69(1): 54-58) and clinical and experimental evidence have also identified damage due to I/R as a risk factor of chronic kidney failure of the allograft.

11. groups of animals were assayed, each one of them formed by 5 rats which were subjected to 60 minutes of left renal ischemia.
1. IR4h C. Saline serum. 4 hours of reperfusion.
2. IR4h CT-1. 400 µg/kg. 4 hours of reperfusion.
3. IR24h C. Saline serum. 24 hours of reperfusion.
4. IR24h CT-1. 400 µg/kg. 24 hours of reperfusion.
5. IR3d C. Saline serum. 3 days of reperfusion.
6. IR3dCT-1. 400 µg/kg. 3 days of reperfusion.
7. IR7d C. Saline serum. 7 days of reperfusion.
8. IR7d CT-1. 400 µg/kg. 7 days of reperfusion.
9. IR14d C. Saline serum. 14 days of reperfusion.
10. IR14d CT-1. 400 µg/kg. 14 days ofreperfusion.
11. Group of simulated rats (Sim).

Thirty minutes after establishment of the ischemia, 500 µl of saline serum (groups IR C) or 400 µg/kg of CT-1 in 500 µl of saline serum (IR CT-1 groups) was administered to the rats through the penile vein. Animals subjected to right nephrectomy were included as simulated group.

### Survival

A survival study was performed with the purpose of examining the protective effect of CT-1 against kidney injury due to I/R. 14 days after the I/R, the untreated ischemic group (IR14d C) showed 40% mortality, whilst in group IR14d CT-1 20% of the animals died (Figure 27).

### Kidney function

48 hours after I/R, the plasma creatinine was markedly higher in animals with IR than in animals from the simulated group (Sim), whilst in group IR CT-1, the plasma creatinine was markedly lower than in IR C, and without significant differences with respect to rats from the simulated group (Sim). (Figure 28A).

48 hours after I/R, the creatinine clearance was markedly less in the IR C group than in the animals from the simulated group, whilst in the IR CT-1 rats it was significantly higher than in IR C animals (Figure 28 B).

### Determination of the production of superoxide anion O₂⁻ (SOA)

Four hours after reestablishment of the blood flow, the renal levels of superoxide anion (SOA) were significantly higher in the ischemic control group (IR C) than in the simulated and IR CT-1 groups. The data from group CT-1 were significantly less than that of the ischemic control group (IR C), but significantly greater than that of the simulated group (Figure 29). Since the infiltration of neutrophils and macrophages is a key stage in I/R-induced tissue inflammation, myeloperoxidase activity (MPO) was measured in renal tissue. The renal levels of myeloperoxidase activity (MA) were more than 5 times greater in the ischemic control group (IR C) than in the simulated group, and were also significantly greater in the ischemic control group than in the CT-1 group. The animals from the IR CT-1 group had similar values to those obtained in the simulated group (Figure 30).

### Determination of the production of proinflammatory cytokines

The ischemic reperfusion induced a great increase in the plasma levels of proinflammatory cytokines 4 hours after reperfusion. The levels of TNF-α (Figure 31A), IL-1β (Figure 31B) and IFN-γ (Figure 31C) were 11, 6 and 4 times greater, respectively, in the IR C group than in the simulated group. Treatment with CT-1 blocked the elevation of these three cytokines induced by IR. Furthermore, the plasma levels of IL-6 (Figure 31D) and IL-10 (Figure 31E) decreased after the ischemia and reperfusion. This decrease was not observed in animals that received CT-1 (Figures 31D and 31E).

### Renal tissue integrity

The histological studies revealed that, 24 hours after reperfusion, the control ischemic kidneys showed abundant cells obstructed by a hyaline residue formed by necrotic tubular cells, as well as by many vacuolated tubular cells. The obstructed tubules and vacuolated cells were less abundant in the kidneys treated with CT-1 than in the control kidneys (Figure 32). 48 hours after reperfusion, the control ischemic kidneys showed many denuded tubules and many areas with inflammatory infiltrate. The denuded tubules and the infiltration was less abundant in the kidneys treated with CT-1 than in the control kidneys (Figure 33).

### CONCLUSIONS

The administration of an anti-CT-1 antibody induces a significant increase in the severity of renal damage in mice subjected to ischemia/reperfusion (I/R), by promoting inflammatory processes, as a result of CT-1 blockade. The results obtained in section A reinforce the role of CT-1 in protecting the kidney from I/R-induced renal damage.

The results obtained allow us to state that recombinant human CT-1 administered intravenously (100 µg/kg/day), in our experimental model of contrast nephropathy in animals sensitized with gentamicin, reduces the deterioration of renal excretory function and the intensity of tubular damage. Furthermore, CT-1 may protect from the vascular and glomerular effects involved in the contrast-induced nephrotoxicity.

As regards the study of acute nephrotoxicity due to drugs, and specifically due to antineoplastic drugs such as cisplatin, the results allow us to conclude that:
1. Treatment with cisplatin, at a single dose of 6 mg/kg intraperitoneally, in male Wistar rats is a suitable *in vivo* experimental model for the study of acute nephrotoxicity due to drugs.
2. The administration of rat cardiotrophin-1, at a dose of 100 µg/kg/day intravenously during 7 days, exercises a protective effect of the cisplatin-induced kidney injury at a dose of 6 mg/kg.

As regards the study of acute nephrotoxicity induced by gentamicin, the results demonstrate that recombinant human CT-1 administered intravenously (100 µg/kg/day, during 7 days -one day before starting treatment with G and throughout it-) has a protective effect on acute kidney injury produced by gentamicin in the Wistar rats. CT-1 reduces deterioration of the renal excretory function and the intensity of the gentamicin-induced renal tubular damage.

As regards the model of acute kidney injury induced by ischemia/reperfusion, the administration of CT-1 prevents acute kidney injury, decrease in kidney function, oxidative stress and ischemia/ reperfusion-induced inflammation.

## Claims

1. A compound which induces cardiotrophin-1 activity (CT-1) for use in the prevention and/or treatment of acute kidney injury, wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

2. A compound for use according to claim 1, wherein the acute kidney injury is selected from the group of acute kidney injury of pre-renal cause and acute kidney injury of renal cause.

3. A compound for use according to claim 2, wherein the acute kidney injury is of pre-renal cause.

4. A compound for use according to claim 3, wherein the acute kidney injury of pre-renal cause is selected from acute kidney injury caused by exposure of a subject to ischemia and acute kidney injury caused by ischemia followed by reperfusion.

5. A compound for use according to claim 2, wherein the acute kidney injury is of renal cause.

6. A compound for use according to claim 5, wherein the acute kidney injury of renal cause is caused by a nephrotoxic agent.

7. A compound for use according to claim 6 wherein the nephrotoxic agent is selected from the group of contrast agents, antibiotics, immunosuppressive agents and antineoplastic agents.

8. A compound for use according to claim 7, wherein the contrast agent is selected from the group of a contrast agent containing iodine, a contrast agent containing bromine, a contrast agent containing barium, a contrast agent containing gadolinium and combinations thereof.

9. A compound for use according to claim 8, wherein the contrast agent contains iodine.

10. A compound for use according to claim 9, wherein the contrast agent containing iodine is selected from the group of diatrizoic acid and its salts, metrizoic acid and its salts, ioxaglic acid and its salts, iothalamic acid and its salts, iopamidol, iohexol, ioxilan, iopromide, ioversol, iodixanol, metrizamide and combinations thereof.

11. A compound for use according to claim 10, wherein the contrast agent containing iodine is one or more diatrizoate salts.

12. A compound for use according to claim 11, wherein the contrast agent containing iodine is a combination of diatrizoate sodium and diatrizoate meglumine.

13. A compound for use according to claim 7, wherein the antibiotic is selected from the group of aminoglycosides and cephalosporins.

14. A compound for use according to claim 13, wherein the aminoglycoside antibiotic is selected from the group of gentamicin, tobramycin, amikacin, netilmicin, kanamycin, streptomycin, sisomycin, neomycin and combinations thereof.

15. A compound for use according to claim 14, wherein the aminoglycoside antibiotic is gentamicin.

16. A compound for use according to claim 7, wherein the immunosuppressive agent is selected from cyclosporin A, tacrolimus (FK506) and everolimus.

17. A compound for use according to claim 7, wherein the antineoplastic agent is selected from the group of a platinum-based compound, an antimetabolite, a DNA alkylating agent, a topoisomerase I or II inhibitor, and a combination thereof.

18. A compound for use according to claim 17, wherein the platinum-based compound is selected from the group of cisplatin, carboplatin and a combination thereof.

19. A compound for use according to claim 18, wherein the compound is cisplatin.

20. A compound for use according to claim 17, wherein the antimetabolite is selected from the group of methotrexate, pentostatin, 5-azacytidine, hydroxyurea and a combination thereof.

21. A compound for use according to claim 17, wherein the DNA alkylating agent is selected from the group of carmustine, lomustine, semustine, ifosfamide, mitomycin C and a combination thereof.

22. A compound for use according to claim 17, wherein the topoisomerase I or II inhibitor is selected from the group of etoposide, teniposide, doxorubicin and a combination thereof.

23. A compound for use according to any of claims 6 to 22, wherein the compound which induces cardiotrophin-1 activity is co-administered together with the nephrotoxic agent.

24. A compound for use according to any of claims 1 to 23 wherein the compound which induces cardiotrophin-1 activity is administered by intravenous route.

25. A compound for use according to any of the preceding claims, wherein the compound which induces cardiotrophin-1 activity is the CT-1 of human origin of SEQ ID NO: 1.

26. A compound for use according to claim 25, wherein cardiotrophin-1 is administered at a dose of 100 µg/kg per day.

27. A compound for use according to any of the preceding claims, wherein the compound which induces cardiotrophin-1 activity reduces the deterioration of the renal excretory function.

28. A compound for use according to any of the preceding claims, wherein the administration of the compound which induces cardiotrophin-1 activity is performed in the absence of a soluble receptor of a member of the IL-6 family or a fragment thereof.

29. A compound for use according to claim 28, where the soluble receptor is the receptor of CT-1 or a fragment thereof.

30. A compound for use according to any of the preceding claims, wherein the administration of the compound which induces cardiotrophin-1 activity is performed in the absence of a metanephric mesenchymal growth factor.

31. A composition comprising, together or separately, a compound which induces cardiotrophin-1 activity and a nephrotoxic agent.

32. A composition according to claim 31, wherein the compound which induces cardiotrophin-1 activity is selected from the group of:
(i) cardiotrophin-1 (CT-1) or a functionally equivalent variant thereof which has, at least, 60% identity with CT-1,
(ii) a polynucleotide which codes for CT-1 or a functionally equivalent variant of CT-1 which has, at least, 60% identity with CT-1,
(iii) a vector comprising a polynucleotide according to (ii), and
(iv) a cell capable of secreting into the medium cardiotrophin-1 or a functionally equivalent variant thereof which has, at least, 60% identity with CT-1.

33. A composition according to any of claims 31 or 32, wherein the nephrotoxic agent is selected from the group of contrast agents, antibiotics, immunosuppressive agents and antineoplastic agents.

34. A composition according to claim 33, wherein the contrast agent is selected from the group of a contrast agent containing iodine, a contrast agent containing bromine, a contrast agent containing barium, a contrast agent containing gadolinium and combinations thereof.

35. A composition according to claim 34, wherein the contrast agent contains iodine.

36. A composition according to claim 35, wherein the contrast agent containing iodine is selected from the group of diatrizoic acid and its salts, metrizoic acid and its salts, ioxaglic acid and its salts, iothalamic acid and its salts, iopamidol, iohexol, ioxilan, iopromide, ioversol, iodixanol, metrizamide and combinations thereof.

37. A composition according to claim 36, wherein the contrast agent containing iodine is one or more diatrizoate salts.

38. A composition according to claim 37, wherein the contrast agent containing iodine is a combination of diatrizoate sodium and diatrizoate meglumine.

39. A composition according to claim 33, wherein the antibiotic is selected from the group of aminoglycosides and cephalosporins.

40. A composition according to claim 39, wherein the aminoglycoside antibiotic is selected from the group of gentamicin, tobramycin, amikacin, netilmicin, kanamycin, streptomycin, sisomycin, neomycin and combinations thereof.

41. A composition according to claim 40, wherein the aminoglycoside antibiotic is gentamicin.

42. A composition according to claim 33, wherein the immunosuppressive agent is selected from cyclosporin A, tacrolimus (FK506) and everolimus.

43. A composition according to claim 33, wherein the antineoplastic agent is selected from the group of a platinum-based compound, an antimetabolite, a DNA alkylating agent, a topoisomerase I or II inhibitor, and a combination thereof.

44. A composition according to claim 43, wherein the platinum-based compound is selected from the group of cisplatin, carboplatin and a combination thereof.

45. A composition according to claim 44, wherein the compound is cisplatin.

46. A composition according to claim 43, wherein the antimetabolite is selected from the group of methotrexate, pentostatin, 5-azacytidine, hydroxyurea and a combination thereof.

47. A composition according to claim 43, wherein the DNA alkylating agent is selected from the group of carmustine, lomustine, semustine, ifosfamide, mitomycin C and a combination thereof.

48. A composition according to claim 43, wherein the topoisomerase I or II inhibitor is selected from the group of etoposide, teniposide, doxorubicin and a combination thereof.

49. A composition according to any of claims 31 to 48, wherein the composition is administered simultaneously, separately or sequentially.

50. A composition according to any of claims 31 to 49, wherein the compound which induces cardiotrophin-1 activity is administered by intravenous route.

51. A composition according to any of claims 31 to 50, wherein the compound which induces cardiotrophin-1 activity is the CT-1 of human origin of SEQ ID NO: 1.

52. A composition according to any of claims 31 to 51, wherein the compound which induces cardiotrophin-1 activity reduces the deterioration of the renal excretory function.

53. A composition according to any of claims 31 to 52, not comprising a soluble receptor of a member of the IL-6 family or a fragment thereof.

54. A composition according to claim 53, where the soluble receptor is the receptor of CT-1 or a fragment thereof.

55. A composition according to any of claims 31 to 54, not comprising a metanephric mesenchymal growth factor.

56. A composition comprising a compound which induces cardiotrophin-1 activity and a contrast agent for use as a diagnostic agent.

57. A composition comprising a compound which induces cardiotrophin-1 activity and an antibiotic for use in the prevention and/or treatment of antibiotic-associated nephrotoxicity.

58. A composition comprising a compound which induces cardiotrophin-1 activity and an immunosuppressive agent for use in the prevention and/or treatment of immunosuppressive agent-associated nephrotoxicity.

59. A composition comprising a compound which induces cardiotrophin-1 activity and an antineoplastic agent for use in the prevention and/or treatment of antineoplastic agent-associated nephrotoxicity.

60. Use of a composition comprising a compound which induces cardiotrophin-1 activity and a contrast agent for the preparation of a diagnostic agent.


SEQUENCE LISTING

<110>  DIGNA Biotech S.L.
       Universidad de Salamanca

<120>  USE OF CARDIOTROPHIN-1 FOR THE TREATMENT OF KIDNEY DISEASES

<130>  P7042PC00

<150>  ES P201100933
<151>  2011-08-10

<160>  2

<170>  PatentIn version 3.5

<210>  1
<211>  201
<212>  PRT
<213>  Homo sapiens

<400>  1

Met Ser Arg Arg Glu Gly Ser Leu Glu Asp Pro Gln Thr Asp Ser Ser
1               5                   10                  15

Val Ser Leu Leu Pro His Leu Glu Ala Lys Ile Arg Gln Thr His Ser
            20                  25                  30

Leu Ala His Leu Leu Thr Lys Tyr Ala Glu Gln Leu Leu Gln Glu Tyr
        35                  40                  45

Val Gln Leu Gln Gly Asp Pro Phe Gly Leu Pro Ser Phe Ser Pro Pro
    50                  55                  60

Arg Leu Pro Val Ala Gly Leu Ser Ala Pro Ala Pro Ser His Ala Gly
65                  70                  75                  80

Leu Pro Val His Glu Arg Leu Arg Leu Asp Ala Ala Ala Leu Ala Ala
                85                  90                  95

Leu Pro Pro Leu Leu Asp Ala Val Cys Arg Arg Gln Ala Glu Leu Asn
            100                 105                 110

Pro Arg Ala Pro Arg Leu Leu Arg Arg Leu Glu Asp Ala Ala Arg Gln
        115                 120                 125

Ala Arg Ala Leu Gly Ala Ala Val Glu Ala Leu Leu Ala Ala Leu Gly
    130                 135                 140

Ala Ala Asn Arg Gly Pro Arg Ala Glu Pro Pro Ala Ala Thr Ala Ser
145                 150                 155                 160

Ala Ala Ser Ala Thr Gly Val Phe Pro Ala Lys Val Leu Gly Leu Arg
                165                 170                 175

Val Cys Gly Leu Tyr Arg Glu Trp Leu Ser Arg Thr Glu Gly Asp Leu
            180                 185                 190

Gly Gln Leu Leu Pro Gly Gly Ser Ala
        195                 200

<210>  2
<211>  239
<212>  PRT
<213>  Artificial Sequence

<220>
<223>  fusion protein of rat cardiotrophin-1 with an N-terminal
       histidine tag

<400>  2

Met Arg Gly Ser His His His His His His Gly Met Ala Ser Met Thr
1               5                   10                  15

Gly Gly Gln Gln Met Gly Arg Asp Leu Tyr Asp Asp Asp Asp Lys Asp
            20                  25                  30

Arg Trp Gly Ser Met Ser Gln Arg Glu Gly Ser Leu Glu Asp His Gln
        35                  40                  45

Thr Asp Ser Ser Phe Ser Phe Leu Pro His Leu Glu Ala Lys Ile Arg
    50                  55                  60

Gln Thr His Asn Leu Ala Arg Leu Leu Thr Lys Tyr Ala Asp Gln Leu
65                  70                  75                  80

Leu Glu Glu Tyr Val Gln Gln Gln Gly Glu Pro Phe Gly Leu Pro Gly
                85                  90                  95

Phe Ser Pro Pro Arg Leu Pro Leu Ala Gly Leu Ser Gly Pro Ala Pro
            100                 105                 110

Ser His Ala Gly Leu Pro Val Ser Glu Arg Leu Arg Gln Asp Ala Ala
        115                 120                 125

Ala Leu Ser Ala Leu Pro Ala Leu Leu Asp Ala Val Arg Arg Arg Gln
    130                 135                 140

Ala Glu Leu Asn Pro Arg Ala Pro Arg Leu Leu Arg Ser Leu Glu Asp
145                 150                 155                 160

Ala Ala Arg Gln Val Arg Ala Leu Gly Ala Ala Val Glu Thr Val Leu
                165                 170                 175

Ala Ala Leu Gly Ala Ala Ala Arg Gly Pro Val Pro Glu Pro Val Ala
            180                 185                 190

Thr Ser Ala Leu Phe Thr Ser Asn Ser Ala Ala Gly Val Phe Ser Ala
        195                 200                 205

Lys Val Leu Gly Leu His Val Cys Gly Leu Tyr Gly Glu Trp Val Ser
    210                 215                 220

Arg Thr Glu Gly Asp Leu Gly Gln Leu Val Pro Gly Gly Val Ala
225                 230                 235